# EUROPÄISCHE PATENTSCHRIFT

(11) **EP 1 917 353 B1**
(45) Veröffentlichungstag und Bekanntmachung des Hinweises auf die Patenterteilung: **08.06.2011**
(21) Anmeldenummer: 06775899.5
(22) Anmeldetag: 23.08.2006
(51) Int. Cl.: C12N 9/50

(54) **VERFAHREN UND MITTEL ZUR ANREICHERUNG, ENTFERNUNG UND ZUM NACHWEIS VON LISTERIEN**
METHOD AND MEANS FOR ENRICHMENT REMOVAL AND DETECTION OF LISTERIA
PROCEDE ET DISPOSITIF D'ENRICHISSEMENT EN LISTERIA, DE SUPPRESSION DE LISTERIA ET DE DETECTION DE LISTERIA

(30) Priorität: 25.08.2005 DE 102005040347
(43) Veröffentlichungstag der Anmeldung: 07.05.2008
(73) Patentinhaber: Biomérieux S.A., 69280 Marcy L'Etoile (FR)
(72) Erfinder: SCHÜTZ, Michael, 93059 Regensburg (DE); OELSCHNER, Maxi, 93051 Regensburg (DE)
(74) Vertreter: Dehmel, Albrecht
(86) Internationale Anmeldenummer: PCT/DE2006/001480
(87) Internationale Veröffentlichungsnummer: WO 2007/022768

(56) Entgegenhaltungen:
- WO-A-00/11472
- US-A1- 2004 197 833
- LOESSNER M J ET AL: "HETEROGENEOUS ENDOLYSINS IN LISTERIA MONOCYTOGENES BACTERIOPHAGES: A NEW CLASS OF ENZYMES AND EVIDENCE FOR CONSERVED HOLIN GENES WITHIN THE SIPHOVIRAL LYSIS CASSETTES" MOLECULAR MICROBIOLOGY, BLACKWELL SCIENTIFIC, OXFORD, GB, Bd. 16, Nr. 6, 1995, Seiten 1231-1241, XP000992666 ISSN: 0950-382X & DATABASE UniProt gefunden im EBI Database accession no. Q38653 & DATABASE EMBL gefunden im EBI Database accession no. X85010
- LOESSNER MARTIN J ET AL: "C-terminal domains of Listeria monocytogenes bacteriophage murein hydrolases determine specific recognition and high-affinity binding to bacterial cell wall carbohydrates" MOLECULAR MICROBIOLOGY, BLACKWELL SCIENTIFIC, OXFORD, GB, Bd. 44, Nr. 2, April 2002 (2002-04), Seiten 335-349, XP002291418 ISSN: 0950-382X
- GAENG S ET AL: "Gene cloning and expression and secretion of Listeria monocytogenes bacteriophage-lytic enzymes in Lactococcus lactis" APPLIED AND ENVIRONMENTAL MICROBIOLOGY, WASHINGTON,DC, US, Bd. 66, Nr. 7, Juli 2000 (2000-07), Seiten 2951-2958, XP001156145 ISSN: 0099-2240

### SEQUENCE LISTING

<110> PROFOS AG
<120> Verfahren und Mittel zur Anreicherung, Entfernung und zum Nachweis von Listerien
<130> PRO-021 PCT
<140> unknown
   <141> 2006-08-23
<150> DE 10 2005 040 347.6
   <151> 2005-08-25
<160> 8
<170> PatentIn version 3.3
<210> 1
   <211> 1026
   <212> DNA
   <213> Phage A511 Endolysin Ply511
<400> 1
   atggtaaaat ataccgtaga gaacaaaatt attgcaggat tacctaaagg taaactaaaa     60
   ggggctaact ttgttattgc tcatgaaact gcaaatagca agtctactat tgacaatgaa    120
   gtaagctaca tgactaggaa ctggaagaac gcatttgtaa ctcactttgt aggtggcgga    180
   ggtagagtcg ttcaggttgc taatgtaaac tatgtttctt ggggagcagg tcagtatgct    240
   aactcttatt cctatgcgca ggtagagttg tgccgtacaa gtaatgcaac tacatttaag    300
   aaagactatg aagtgtactg tcaattacta gtagacctag ctaaaaaagc aggtatccct    360
   attacacttg actctggtag taaaactagt gataaaggta ttaaatccca taaatgggtt    420
   gctgataagc taggaggaac aacacaccaa gacccatatg cttacttaag ctcatggggt    480
   attagtaaag cacaatttgc tagtgacttg gctaaagtat ctggcggagg aaacacagga    540
   acagcgccag ctaaaccaag cacaccagca cctaaaccaa gcacaccatc tactaaccta    600
   gacaaacttg gcttagtaga ctacatgaac gctaagaaaa tggactctag ctacagtaac    660
   agagataagt tagctaaaca gtatggtatt gctaactatt caggaacagc tagccagaac    720
   actacactcc ttagtaaaat taaaggagga gcacctaaac caagcacacc agcacctaaa    780
   cctagtacat ctacagctaa gaaaatttat ttcccaccaa ataaaggaaa ctggtctgtg    840
   tatccaacaa ataaagcacc cgttaaggct aatgctattg gtgctattaa ccctactaaa    900
   ttcggaggat tgacttacac tatccaaaaa gatagaggaa acggtgtata cgaaatccaa    960
   acagaccaat tcggcagagt tcaagtctat ggtgcaccta gtacaggagc agttatcaaa   1020
   aaataa                                                              1026
<210> 2
   <211> 341
   <212> PRT
   <213> Phage A511 Endolysin Ply511
<400> 2
   Met Val Lys Tyr Thr Val Glu Asn Lys Ile Ile Ala Gly Leu Pro Lys
   1               5                   10                  15
   Gly Lys Leu Lys Gly Ala Asn Phe Val Ile Ala His Glu Thr Ala Asn
               20                  25                  30
   Ser Lys Ser Thr Ile Asp Asn Glu Val Ser Tyr Met Thr Arg Asn Trp
           35                  40                  45
   Lys Asn Ala Phe Val Thr His Phe Val Gly Gly Gly Gly Arg Val Val
       50                  55                  60
   Gln Val Ala Asn Val Asn Tyr Val Ser Trp Gly Ala Gly Gln Tyr Ala
   65                  70                  75                  80
   Asn Ser Tyr Ser Tyr Ala Gln Val Glu Leu Cys Arg Thr Ser Asn Ala
                   85                  90                  95
   Thr Thr Phe Lys Lys Asp Tyr Glu Val Tyr Cys Gln Leu Leu Val Asp
               100                 105                 110
   Leu Ala Lys Lys Ala Gly Ile Pro Ile Thr Leu Asp Ser Gly Ser Lys
           115                 120                 125
   Thr Ser Asp Lys Gly Ile Lys Ser His Lys Trp Val Ala Asp Lys Leu
       130                 135                 140
   Gly Gly Thr Thr His Gln Asp Pro Tyr Ala Tyr Leu Ser Ser Trp Gly
   145                 150                 155                 160
   Ile Ser Lys Ala Gln Phe Ala Ser Asp Leu Ala Lys Val Ser Gly Gly
                   165                 170                 175
   Gly Asn Thr Gly Thr Ala Pro Ala Lys Pro Ser Thr Pro Ala Pro Lys
               180                 185                 190
   Pro Ser Thr Pro Ser Thr Asn Leu Asp Lys Leu Gly Leu Val Asp Tyr
           195                 200                 205
   Met Asn Ala Lys Lys Met Asp Ser Ser Tyr Ser Asn Arg Asp Lys Leu
       210                 215                 220
   Ala Lys Gln Tyr Gly Ile Ala Asn Tyr Ser Gly Thr Ala Ser Gln Asn
   225                 230                 235                 240
   Thr Thr Leu Leu Ser Lys Ile Lys Gly Gly Ala Pro Lys Pro Ser Thr
                   245                 250                 255
   Pro Ala Pro Lys Pro Ser Thr Ser Thr Ala Lys Lys Ile Tyr Phe Pro
               260                 265                 270
   Pro Asn Lys Gly Asn Trp Ser Val Tyr Pro Thr Asn Lys Ala Pro Val
           275                 280                 285
   Lys Ala Asn Ala Ile Gly Ala Ile Asn Pro Thr Lys Phe Gly Gly Leu
       290                 295                 300
   Thr Tyr Thr Ile Gln Lys Asp Arg Gly Asn Gly Val Tyr Glu Ile Gln
   305                 310                 315                 320
   Thr Asp Gln Phe Gly Arg Val Gln Val Tyr Gly Ala Pro Ser Thr Gly
                   325                 330                 335
   Ala Val Ile Lys Lys
               340
<210> 3
   <211> 681
   <212> DNA
   <213> Phage A511 Endolysin Ply511
<400> 3
   aaagcaggta tccctattac acttgactct ggtagtaaaa ctagtgataa aggtattaaa      60
   tcccataaat gggttgctga taagctagga ggaacaacac accaagaccc atatgcttac     120
   ttaagctcat ggggtattag taaagcacaa tttgctagtg acttggctaa agtatctggc     180
   ggaggaaaca caggaacagc gccagctaaa ccaagcacac cagcacctaa accaagcaca     240
   ccatctacta acctagacaa acttggctta gtagactaca tgaacgctaa gaaaatggac     300
   tctagctaca gtaacagaga taagttagct aaacagtatg gtattgctaa ctattcagga     360
   acagctagcc agaacactac actccttagt aaaattaaag gaggagcacc taaaccaagc     420
   acaccagcac ctaaacctag tacatctaca gctaagaaaa tttatttccc accaaataaa     480
   ggaaactggt ctgtgtatcc aacaaataaa gcacccgtta aggctaatgc tattggtgct     540
   attaacccta ctaaattcgg aggattgact tacactatcc aaaaagatag aggaaacggt     600
   gtatacgaaa tccaaacaga ccaattcggc agagttcaag tctatggtgc acctagtaca     660
   ggagcagtta tcaaaaaata a                                               681
<210> 4
   <211> 226
   <212> PRT
   <213> Phage A511 Endolysin Ply511
<400> 4
   Lys Ala Gly Ile Pro Ile Thr Leu Asp Ser Gly Ser Lys Thr Ser Asp
   1               5                   10                  15
   Lys Gly Ile Lys Ser His Lys Trp Val Ala Asp Lys Leu Gly Gly Thr
               20                  25                  30
   Thr His Gln Asp Pro Tyr Ala Tyr Leu Ser Ser Trp Gly Ile Ser Lys
           35                  40                  45
   Ala Gln Phe Ala Ser Asp Leu Ala Lys Val Ser Gly Gly Gly Asn Thr
       50                  55                  60
   Gly Thr Ala Pro Ala Lys Pro Ser Thr Pro Ala Pro Lys Pro Ser Thr
   65                  70                  75                  80
   Pro Ser Thr Asn Leu Asp Lys Leu Gly Leu Val Asp Tyr Met Asn Ala
                   85                  90                  95
   Lys Lys Met Asp Ser Ser Tyr Ser Asn Arg Asp Lys Leu Ala Lys Gln
               100                 105                 110
   Tyr Gly Ile Ala Asn Tyr Ser Gly Thr Ala Ser Gln Asn Thr Thr Leu
           115                 120                 125
   Leu Ser Lys Ile Lys Gly Gly Ala Pro Lys Pro Ser Thr Pro Ala Pro
       130                 135                 140
   Lys Pro Ser Thr Ser Thr Ala Lys Lys Ile Tyr Phe Pro Pro Asn Lys
   145                 150                 155                 160
   Gly Asn Trp Ser Val Tyr Pro Thr Asn Lys Ala Pro Val Lys Ala Asn
                   165                 170                 175
   Ala Ile Gly Ala Ile Asn Pro Thr Lys Phe Gly Gly Leu Thr Tyr Thr
               180                 185                 190
   Ile Gln Lys Asp Arg Gly Asn Gly Val Tyr Glu Ile Gln Thr Asp Gln
           195                 200                 205
   Phe Gly Arg Val Gln Val Tyr Gly Ala Pro Ser Thr Gly Ala Val Ile
       210                 215                 220
   Lys Lys
   225
<210> 5
   <211> 594
   <212> DNA
   <213> Phage A511 Endolysin Ply511
<400> 5
   ggaggaacaa cacaccaaga cccatatgct tacttaagct catggggtat tagtaaagca     60
   caatttgcta gtgacttggc taaagtatct ggcggaggaa acacaggaac agcgccagct    120
   aaaccaagca caccagcacc taaaccaagc acaccatcta ctaacctaga caaacttggc    180
   ttagtagact acatgaacgc taagaaaatg gactctagct acagtaacag agataagtta    240
   gctaaacagt atggtattgc taactattca ggaacagcta gccagaacac tacactcctt    300
   agtaaaatta aaggaggagc acctaaacca agcacaccag cacctaaacc tagtacatct    360
   acagctaaga aaatttattt cccaccaaat aaaggaaact ggtctgtgta tccaacaaat    420
   aaagcacccg ttaaggctaa tgctattggt gctattaacc ctactaaatt cggaggattg    480
   acttacacta tccaaaaaga tagaggaaac ggtgtatacg aaatccaaac agaccaattc    540
   ggcagagttc aagtctatgg tgcacctagt acaggagcag ttatcaaaaa ataa          594
<210> 6
   <211> 197
   <212> PRT
   <213> Phage A511 Endolysin Ply511
<400> 6
   Gly Gly Thr Thr His Gln Asp Pro Tyr Ala Tyr Leu Ser Ser Trp Gly
   1               5                   10                  15
   Ile Ser Lys Ala Gln Phe Ala Ser Asp Leu Ala Lys Val Ser Gly Gly
               20                  25                  30
   Gly Asn Thr Gly Thr Ala Pro Ala Lys Pro Ser Thr Pro Ala Pro Lys
           35                  40                  45
   Pro Ser Thr Pro Ser Thr Asn Leu Asp Lys Leu Gly Leu Val Asp Tyr
       50                  55                  60
   Met Asn Ala Lys Lys Met Asp Ser Ser Tyr Ser Asn Arg Asp Lys Leu
   65                  70                  75                  80
   Ala Lys Gln Tyr Gly Ile Ala Asn Tyr Ser Gly Thr Ala Ser Gln Asn
                   85                  90                  95
   Thr Thr Leu Leu Ser Lys Ile Lys Gly Gly Ala Pro Lys Pro Ser Thr
               100                 105                 110
   Pro Ala Pro Lys Pro Ser Thr Ser Thr Ala Lys Lys Ile Tyr Phe Pro
           115                 120                 125
   Pro Asn Lys Gly Asn Trp Ser Val Tyr Pro Thr Asn Lys Ala Pro Val
       130                 135                 140
   Lys Ala Asn Ala Ile Gly Ala Ile Asn Pro Thr Lys Phe Gly Gly Leu
   145                 150                 155                 160
   Thr Tyr Thr Ile Gln Lys Asp Arg Gly Asn Gly Val Tyr Glu Ile Gln
                   165                 170                 175
   Thr Asp Gln Phe Gly Arg Val Gln Val Tyr Gly Ala Pro Ser Thr Gly
               180                 185                 190
   Ala Val Ile Lys Lys
           195
<210> 7
   <211> 528
   <212> DNA
   <213> Phage A511 Endolysin Ply511
<400> 7
   gctagtgact tggctaaagt atctggcgga ggaaacacag gaacagcgcc agctaaacca     60
   agcacaccag cacctaaacc aagcacacca tctactaacc tagacaaact tggcttagta    120
   gactacatga acgctaagaa aatggactct agctacagta acagagataa gttagctaaa    180
   cagtatggta ttgctaacta ttcaggaaca gctagccaga acactacact ccttagtaaa    240
   attaaaggag gagcacctaa accaagcaca ccagcaccta aacctagtac atctacagct    300
   aagaaaattt atttcccacc aaataaagga aactggtctg tgtatccaac aaataaagca    360
   cccgttaagg ctaatgctat tggtgctatt aaccctacta aattcggagg attgacttac    420
   actatccaaa aagatagagg aaacggtgta tacgaaatcc aaacagacca attcggcaga    480
   gttcaagtct atggtgcacc tagtacagga gcagttatca aaaaataa                528
<210> 8
   <211> 175
   <212> PRT
   <213> Phage A511 Endolysin Ply511
<400> 8
   Ala Ser Asp Leu Ala Lys Val Ser Gly Gly Gly Asn Thr Gly Thr Ala
   1               5                   10                  15
   Pro Ala Lys Pro Ser Thr Pro Ala Pro Lys Pro Ser Thr Pro Ser Thr
               20                  25                  30
   Asn Leu Asp Lys Leu Gly Leu Val Asp Tyr Met Asn Ala Lys Lys Met
           35                  40                  45
   Asp Ser Ser Tyr Ser Asn Arg Asp Lys Leu Ala Lys Gln Tyr Gly Ile
       50                  55                  60
   Ala Asn Tyr Ser Gly Thr Ala Ser Gln Asn Thr Thr Leu Leu Ser Lys
   65                  70                  75                  80
   Ile Lys Gly Gly Ala Pro Lys Pro Ser Thr Pro Ala Pro Lys Pro Ser
                   85                  90                  95
   Thr Ser Thr Ala Lys Lys Ile Tyr Phe Pro Pro Asn Lys Gly Asn Trp
               100                 105                 110
   Ser Val Tyr Pro Thr Asn Lys Ala Pro Val Lys Ala Asn Ala Ile Gly
           115                 120                 125
   Ala Ile Asn Pro Thr Lys Phe Gly Gly Leu Thr Tyr Thr Ile Gln Lys
       130                 135                 140
   Asp Arg Gly Asn Gly Val Tyr Glu Ile Gln Thr Asp Gln Phe Gly Arg
   145                 150                 155                 160
   Val Gln Val Tyr Gly Ala Pro Ser Thr Gly Ala Val Ile Lys Lys
                   165                 170                 175

## Beschreibung

Die vorliegende Erfindung betrifft Polypeptidfragmente von Endolysin Ply511, die Listerien Serotyp unabhängig erkennen und binden, aber keine Zellwand hydrolysierende enzymatische Aktivität aufweisen. Die Erfindung betrifft ferner Verfahren zur Anreicherung, Entfernung und zum Nachweis von Listerien.

Listerien sind human- und tierpathogene Bakterien, die häufig in Nahrungsmitteln insbesondere in Fisch, Fleisch und Milchprodukten vorkommen. Die Gattung Listeria umfaßt 6 verschiedene Species mit 16 unterschiedlichen Serotypen. Im Einzelnen sind dies *L. monocytogenes* mit den Serotypen 1/2a, 1/2b, 1/2c, 3a, 3b, 3c, 4a, 4ab, 4b, 4c, 4d, 4e, 7; *L. innocua* mit den Serotypen 3, 6a, 6b, 4ab, U/S; *L. ivanovii* mit dem Serotyp 5; *L. seeligeri* mit den Serotypen 1/2a, 1/2b, 1/2c, 4b, 4c, 4d, 6b; *L. welshimeri* mit den Serotypen 1/2a, 4c, 6a, 6b,U/S und *L. grayi* mit dem Serotyp Grayi. Die beiden Species *L. monocytogenes* und *L. ivanovii* gelten als pathogen. Eine dritte Species, *L. seeligeri,* wird als apathogen betrachtet, jedoch ist ein Fall bekannt, bei dem *L. seeligeri* bei einem Menschen Meningitis verursachte. Die übrigen Species gelten als apathogen. Ca. 90% der Listeriosen werden auf *L. monocytogenes* Serovar 1/2a, 1/2b und 4b zurückgeführt (Wing EJ & Gregory SH, 2002, Listeria monocytogenes: Clinical and Experimental Update, J Infect Diseases 185 (Suppl 1): S18-S24).

Obwohl nur ein geringer Anteil der nahrungsmittelbedingten Erkrankungen von *Listerien* ausgelöst werden (ca. 1% in den USA), werden nahezu 30 % der jährlich tödlich verlaufenden Erkrankungen, die durch Nahrungsmittelpathogene verursacht werden, diesem Keim zugeschrieben. Betroffen sind vor allem immunsupprimierte Personen, z.B. ältere Menschen, Diabetiker, an Krebs und/oder AIDS erkrankte Personen. Schwangere und das noch ungeborene Kind stellen ca. 25 % aller Fälle an invasiven Listeriosen erkrankten dar. Aufgrund ihrer Fähigkeit die Blut-Hirnschranke oder die Placentaschranke zu überwinden, können Listerien Meningitis, Encephalitis, Abgänge und Todgeburten verursachen (Wing EJ & Gregory SH, 2002, Listeria monocytogenes: Clinical and Experimental Update, J Infect Deseases 185 (Suppl 1): S18-S24; Doyle ME, 2001, Virulence Characteristics of Listeria monocytogenes, Food Research Institute, October 2001).

Listerien sind sehr tolerant gegenüber leichten Säuren und sind in der Lage, sich bei relativ hohen Salzkonzentrationen und bei Temperaturen von 1 °C bis 45 °C zu vermehren. Die hauptsächliche Infektionsquelle sind Nahrungsmittel, insbesondere diejenigen, die vor dem Verzehr nicht hitzebehandelt werden, wie z.B. viele Milchprodukte, Räucherfisch, Fleischwaren und in zunehmenden Maße auch Fertigprodukte (ready-to-eat-Produkte). Während in den USA eine Nulltoleranz für *L. monocytogenes* in genussfertigen Lebensmitteln gilt, ist in vielen europäische Länder oder auch Kanada für bestimmte Nahrungsmittel eine Kontamination mit Listerien bis zu 100 KbE (Kolonie-bildende Einheiten) / g Nahrungsmittel zulässig. In jedem Fall müssen jedoch die Nahrungsmittel auf Listerienkontamination hin untersucht werden. Viele dieser Nahrungsmittel, z.B. Meeresfrüchte, Räucherlachs, Milchprodukte oder auch Rohkostfertigprodukte haben nur eine geringe Haltbarkeit. So kommt es häufig zu kostenintensiven Rückrufaktionen, wenn bei diesen Produkten nach Auslieferung eine Listerienkontamination bzw. eine Kontamination über dem erlaubten Grenzwert festgestellt wurde. Aus diesem Grund besteht großes Interesse, die bisher relativ lange Zeitspanne, die notwendig ist um eine Kontamination nachzuweisen entscheidend zu verkürzen.

Für eine ausreichende Kontrolle der Lebensmittel hinsichtlich Listeria ist der Nachweis von L. monocytogenes allein nicht ausreichend, zumal auch Fälle von Listeriosen ausgelöst durch L. ivanovii und L. seeligeri bekannt sind. Zusätzlich kann eine Prüfung auf alle Listerienarten als Hygienemonitoring bei der Nahrungsmittelproduktion verwendet werden. Ein Problem beim Nachweis von Listerien ist die Zeit, die für Nachweis notwendig ist. Insbesondere in der Nahrungsmittelindustrie stellt die Nachweiszeit einen bedeutenden Faktor im Hinblick auf eine kurze Haltbarkeit mancher Lebensmittel und einer kostspieligen Lagerung dar, die notwendig ist, bis abgesichert ist, dass die Probe nicht kontaminiert ist. Darüber hinaus können immer wieder kostspielige Rückrufaktionen beobachtet werden, wenn kontamierte Ware vor Erhalt der Kontrollergebnisse vorzeitig ausgeliefert wurde. So betragen die Standard Nachweiszeiten nach ISO 11290-1:1996/FDAM 1:2004(E) mehr als 4-7 Tage und nach FDA und USDA/FSIS 4-7 Tage. Für viele andere Methoden dauert die Anreicherung bis zu 48 h, um eine ausreichende Menge an Listerien für den Nachweis und diese ohne störende Lebensmittelprobenbestandteile zu bekommen.

Diesem Problem haben sich eine Vielzahl von Nachweismethoden für Listerien angenommen, wobei viele davon auch kommerziell erhältlich sind, z.B. PCR, Elisa, usw. (siehe US 2004/0197833 A1). Eine derartige Methode ist bei Reinkulturen zwar sehr viel versprechend, weist bei Mischkulturen oder komplexen Matrizes wie Lebensmitteln jedoch erhebliche Probleme auf.

So nutzt ein Verfahren Antikörper gegen die Flagellenproteine von L. monoctogenes (Skjerve, 1990; Skjerve E, Rorvik LM, Olsvik O. Detection of Listeria monocytogenes in foods by immunomagnetic separation. Appl Environ Microbiol. 1990 (11):3478-3481) immobilisiert auf magnetischen Partikeln. Mit einem ähnlichen Verfahren bei Versuchen mit Käse (Uyttendaele et al., 2000; Uyttendaele M, Van Hoorde I, Debevere J., The use of immuno-magnetic separation (IMS) as a tool in a sample preparation method for direct detection of L. monocytogenes in cheese. Int J Food Microbiol. 2000;54(3):205-212.), konnten so Zellzahlen von 0,5-1,5 Kbe pro Gramm Käse detektiert werden. Dabei wurde jedoch festgestellt, dass die Antikörper nicht nur die Zielzellen binden. Eine derartige Methode ist bei Reinkulturen zwar sehr viel versprechend, weist bei Mischkulturen oder komplexen Matrizes wie Lebensmitteln jedoch erhebliche Probleme auf. So war der Anteil an gebundenen Nicht-Listerien sehr hoch. Außerdem konnten nur dann ausreichend Listerien über das Verfahren gewonnen werden, wenn die Nahrungsmittelhomogenate zur Bindung der Zellen an die Partikel verdünnt, zentrifugiert und enzymatisch verdaut wurden. In einem weiteren Versuch (Jung et al., 2003; Jung YS, Frank JF, Brackett RE. Evaluation of antibodies for immunomagnetic separation combined with flow cytometry detection of Listeria monocytogenes. J Food Prot. 2003; 66(7):1283-1287) zeigten Jung et al., dass über die Antikörper-beschichteten magnetischen Beads nur 7% - 23% der Listerien aus einer Pufferlösung isoliert werden konnten.

Wiederfindungsraten von 5% - 15% der eingesetzten Listerien wurden auch in Versuchen von Fluit et al. (Fluit et al.1993; Fluit AC, Torensma R, Visser MJ, Aarsman CJ, Poppelier MJ, Keller BH, Klapwijk P, Verhoef J. Detection of Listeria monocytogenes in cheese with the magnetic immuno-polymerase chain reaction assay. Appl Environ Microbiol. 1993; 59(5):1289-1293) mit magnetischen Partikeln erhalten, die mit monoklonalen Antikörpern gegen ganze Listeria-Zellen ohne Flagellen, sowie monoklonalen Antikörpern gegen das Listerien-Flagellenprotein beschichtet waren. Zusätzlich wurden 10 % der Listerien von den Antikörpern nicht erkannt.

Ein weiteres Verfahren nutzt die zellbindenden Domänen (CBD) zweier Listeriaphagen-Endolysine (WO 00/11472; US2004/0197833 A1). Endolysine sind phagencodierte Proteine die in der späten Phase der Phagenreifung produziert werden und zusammen mit einem membranporenbildenden Holin die Lyse der Wirtszelle zur Freisetzung der reifen Phagenpartikel ermöglicht. Endolysine bestehen aus 2 Domänen, einer lytischen Domäne und einer Zellbindedomäne (CBD). Die lytische Domäne, spaltet das Peptidoglycan der Zellwand Grampositiver Bakterien. Die CBD bindet an die Zellwand und bestimmt die Spezifität des Endolysins.

In diesem Verfahren (WO 00/11472; US2004/0197833 A1) wurden Fusionen aus einem Green-Fluorescence-Protein (GFP) mit den CBDs (CBD118 und CBD500) der Endolysine Ply118 und Ply500 aus den Listerienphagen A118 und A500 verwendet. Diese Proteine wurden an magnetischen Partikeln immobilisiert und auf ihre Fähigkeit zur Anreicherung von Listerien hin untersucht. Das CBD118/CBD500 System wurde mit verschiedenen Nahrungsmittelproben vergleichend zur IDF-Standard-Methode (IDF, 143A:1995) getestet. Es zeigte sich, dass durch den Einsatz der beschichteten Partikel die Voranreicherungsdauer wesentlich verkürzt werden konnte, um Listerien in Nahrungsmitteln nachweisen zu können.

Hinsichtlich der Bindeeigenschaften der CBDs hatte sich jedoch gezeigt, dass sowohl CBD118 als auch CBD500 alleine nicht alle Serotypen erkennen. Daher ist es notwendig, immer eine Mischung mit beiden CBDs gekoppelt an Partikel zur Anreicherung einzusetzen, da nicht von vorneherein bekannt ist, welchen Serotyp bzw. welche Serotypen die Listerien in den Proben aufweisen. Dadurch verdoppelt sich die pro Nahrungsmitteltest einzusetzende Partikelmenge, was zu einer Verteuerung des Systems führt und das Risiko unspezifisch an den Partikeln anhaftender Nicht-Listerien erhöht.

Daher liegt der Erfindung die Aufgabe zu Grunde, ein Verfahren und die Mittel zu seiner Durchführung bereitzustellen, mit denen Listerien aller Serotypen einfach und schnell nachgewiesen und entfernt werden können.

Die Aufgabe wird durch den in den Patentansprüchen definierten Gegenstand gelöst.

Die nachfolgenden Figuren erläutern die Erfindung.

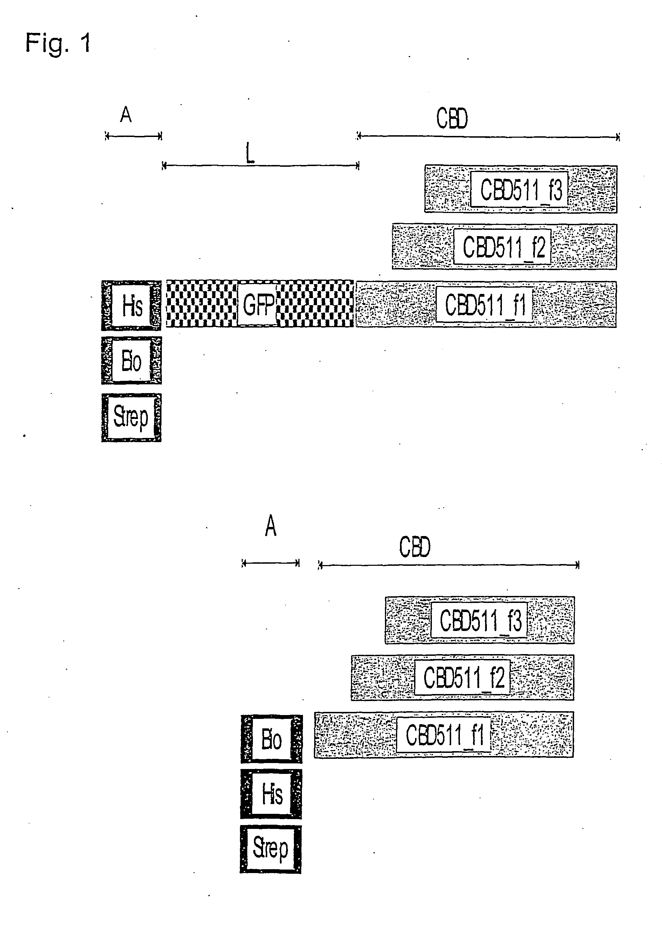

Fig. 1 zeigt schematisch verschiedene Kombinationen von Fusionsproteinen mit Varianten der Zellbindedomäne des Endolysins Ply511. Die Abkürzung A bedeutet Affinitätsdomäne, "L" bedeutet Linkerdomäne und "Z" Zellbindedomäne. Die Abkürzung His bedeutet His-Tag; die Abkürzung Bio steht für eine Domäne die ein Biotinmolekül trägt; Strep steht für Strep-Tag; GFP steht für "green fluoreszent protein" als Linker-Domäne; CBD511 steht für Zellbindedomäne (cell binding domain) des Endolysins Ply511 mit den jeweiligen Varianten _f1, f2 und _f3.

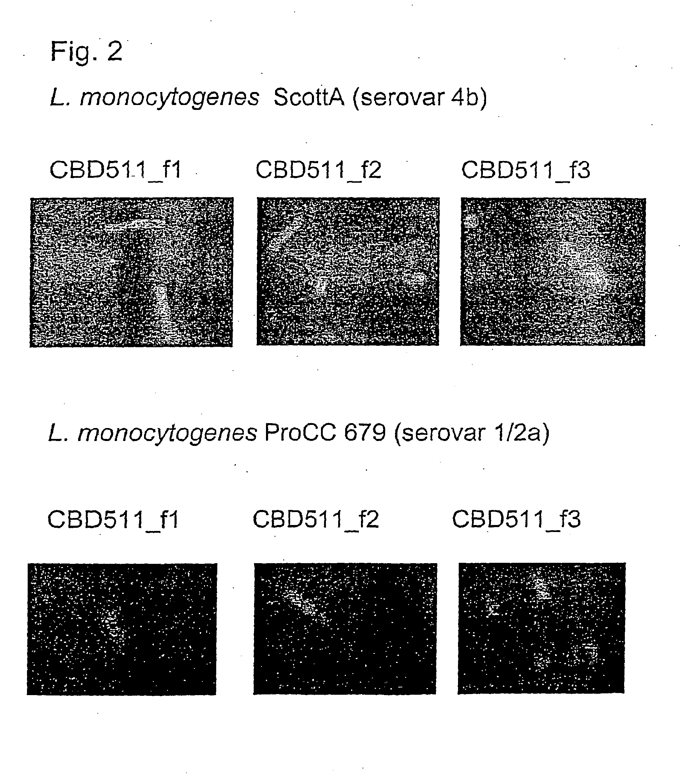

Fig. 2 zeigt fluoreszenzmikroskopische Aufnahmen der Bindung verschiedener GFP-CBD-Fusionen an *L. monocytogenes* ScottA (Serovar 4b) *und L. monocytogenes* ProCC 679 (Serovar 1/2a). Versuchsanordnung siehe Experiment 1. CBD511_f1 steht für die Fusion aus StrepTag-HisTag-GFP-CBD f1, CBD511_f2 steht für die Fusion aus StrepTag-HisTag-GFP-CBD_f2 und CBD511 f3 steht für die Fusion aus StrepTag-HisTag-GFP-CBD511 f3

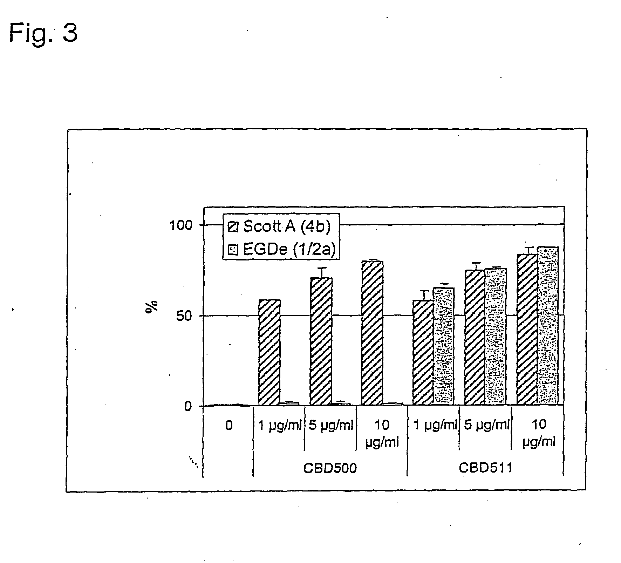

Fig. 3 zeigt die Serotyp-Abhängigkeit der Bindung der Zellbindedomänen CBD500 und CBD511 bei verschiedenen Proteinkonzentrationen. Dargestellt ist die Menge der an die magnetischen Partikel gebundenen Zellen in Prozent (im Vergleich zu der Zahl) der insgesamt wieder gefundenen Zellen. Versuchsdurchführung beschrieben in Experiment 3. CBD500 steht für das Fusionsprotein StrepTag-HisTag-GFP-CBD500; CBD511 steht für das Fusionsprotein StrepTag-HisTag-GFP-CBD511 f2; ScottA (4b) steht für *L. monocytogenes* ScottA mit dem Serotyp 4b; EGDe (1/2a) steht für *L. monocytogenes* EGDe mit dem Serotyp 1/2a. Die Werte sind Mittelwerte aus 8 Versuchen. Die jeweils eingesetzte Zellzahl betrug zwischen 75 und 120 KbE /ml

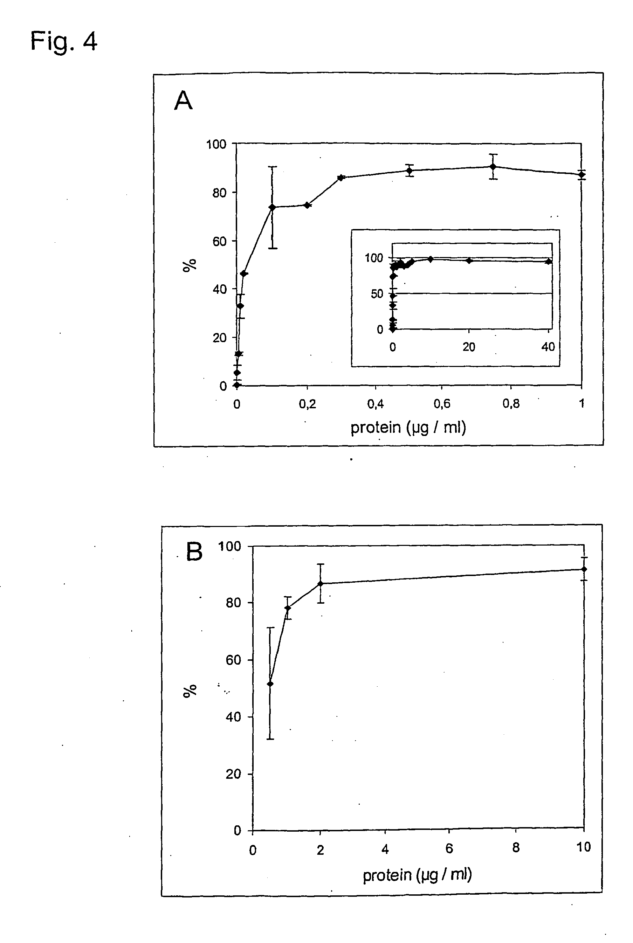

Fig. 4 zeigt die Abhängigkeit der Zellbindung an magnetische Partikel von der Konzentration an biotinylierten Konstrukten der CBD511 - Bio-OD-CBD511_f3 (Fig. 4A) und Bio-Av-GFP-CBD511 f3 (Fig. 4B). Dargestellt ist die Menge der an die magnetischen Partikel gebundenen Zellen in Prozent (im Vergleich zu der Zahl) der insgesamt wieder gefundenen Zellen. Die Versuchsdurchführung ist beschrieben in Experiment 4. Die eingesetzte Zellzahl an *L*. *monocytogenes* ScottA, Serovar 4b, betrug 1 x 10³ - 1 x 10⁴ KbE /ml. Die Datenpunkte wurden ermittelt aus 2-8 unabhängigen Experimenten.

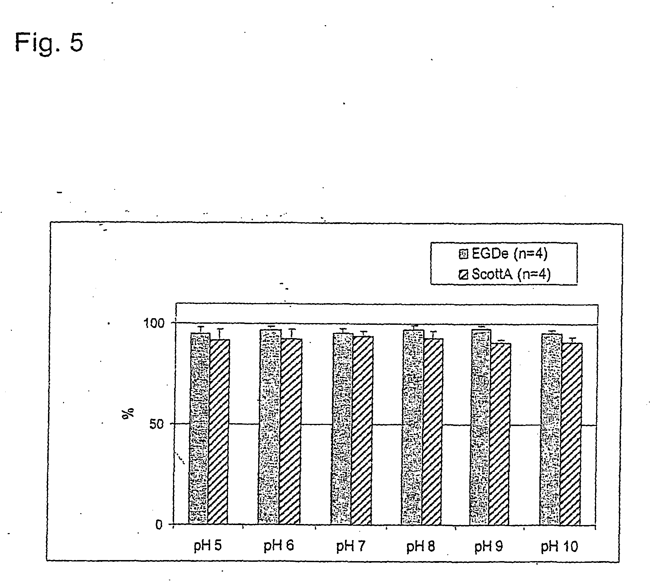

Fig. 5 zeigt die pH-Abhängigkeit der CBD511 vermittelten Bindung von *L. monocytogenes* Serovar 1/2a und 4b aus Lösungen an magnetische Partikel mittels des 2-Schritt Verfahrens. Dargestellt ist die Menge der mit Hilfe des Fusionsproteins Bio-Av-GFP-CBD_f3 an die magnetischen Partikel gebundenen Zellen in Prozent (im Vergleich zu der Zahl) der insgesamt wieder gefundenen Zellen. Versuchsdurchführung beschrieben in Experiment 5. ScottA (4b) steht für *L. monocytogenes* ScottA mit dem Serotyp 4b; EGDe (1/2a) steht für *L. monocytogenes* EGDe mit dem Serotyp 1/2a. Die Werte sind Mittelwerte aus 4 Versuchen. Die jeweils eingesetzte Zellzahl betrug zwischen 10³ bis 10⁴ KbE/ml.

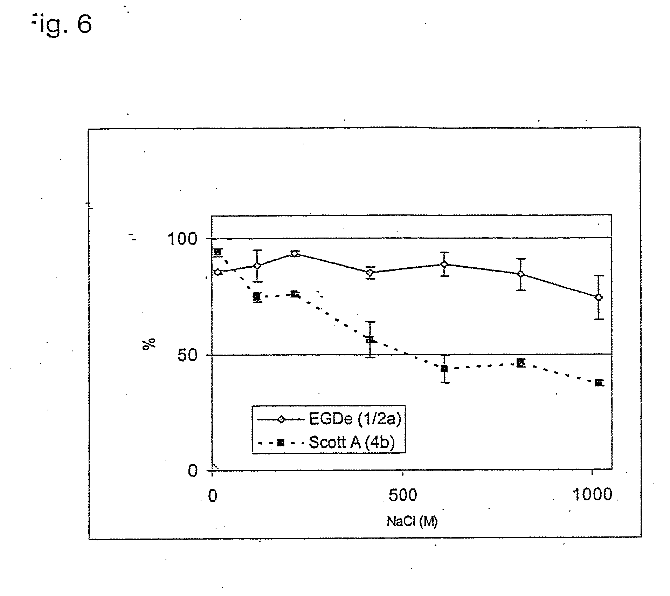

Fig. 6 zeigt die Salzkonzentrationsabhängigkeit der CBD511 vermittelten Bindung von *L. monocytogenes* serovar 1/2a und 4b aus Lösungen an magnetische Partikel mittels des 2-Schritt Verfahrens. Dargestellt ist die Menge der an die magnetischen Partikel gebundenen Zellen in Prozent (im Vergleich zu der Zahl) der insgesamt wieder gefundenen Zellen. Versuchsdurchführung beschrieben in Experiment 6. ScottA (4b) steht für *L. monocytogenes* ScottA mit dem Serotyp 4b; EGDe (1/2a) steht für *L. monocytogenes* EGDe mit dem Serotyp 1/2a. NaCl steht für Natriumchlorid. Die Werte sind Mittelwerte aus 4 Versuchen. Die jeweils eingesetzte Zellzahl betrug ca. 4-8 x 10³ KbE/ml.

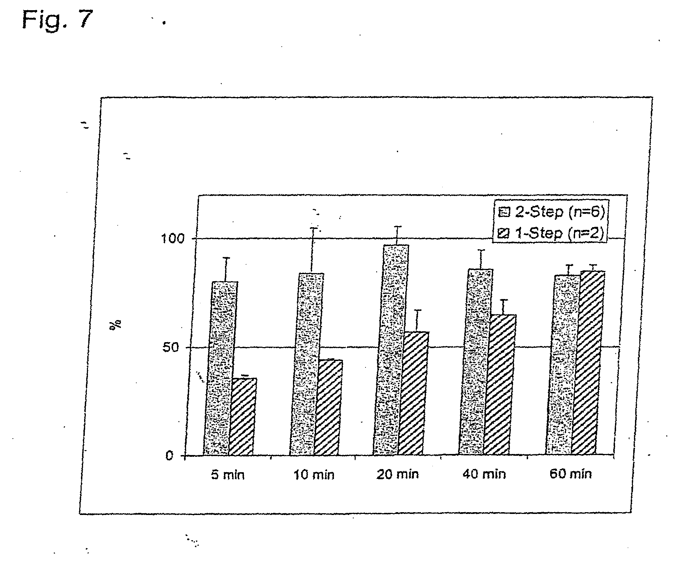

Fig. 7 zeigt die Abhängigkeit der Anzahl der an die magnetischen Partikel gebundenen Listerienzellen von der Inkubationsdauer beim 1-Schritt und beim 2-Schritt Verfahren. Dargestellt ist die Menge der an die magnetischen Partikel gebundenen Zellen in Prozent (im Vergleich zu der Zahl) der insgesamt wieder gefundenen Zellen. (Versuchsdurchführung beschrieben in Experiment 7) 2-Step steht für 2-Schritt Verfahren mit Bio-Av-GFP-CBD f3 (1 µg/ml) und Streptavidin-beschichteten magnetischen Partikeln (50 µg/ml), 1-Step steht für 1-Schritt-Verfahren Zellbindung an 300 µg/ml Dynabeads M-270 Epoxy kovalent beschichtet mit Bio-Av-GFP-CBD_f3. Die jeweils eingesetzte Zellzahl betrug ca. 4-8 x 10³ KbE/ml. Die Ergebnisse sind gemittelt aus 6 Versuchen (2-Schritt Verfahren) bzw. 2 Versuchen (1-Schritt-Verfahren).

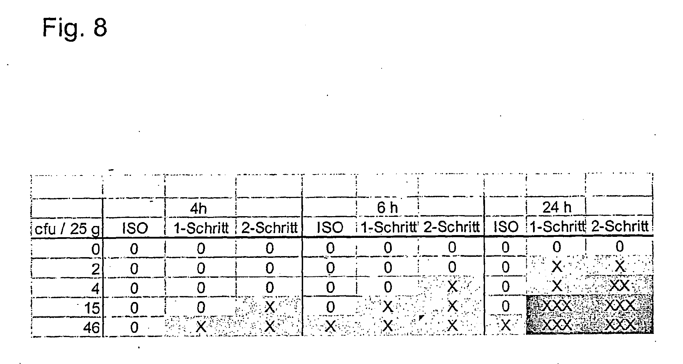

Fig. 8 zeigt im Vergleich von ISO-Norm, 1-Schritt Verfahren und 2-Schritt Verfahren die Zeitabhängigkeit des Nachweises von *L. monocytogenes* in Camembert. Versuchsdurchführung beschrieben in Experiment 7. 5 Portionen Camembert (25 g) wurden mit 0, 2, 4, 15 und 46 KbE kontaminiert, in 225 ml Fraser ½ homogenisiert und bei 30 °C inkubiert. Proben wurden zu den angegebenen Zeiten entnommen und gemäß ISO: 11290-1:1996 FDAM1, nach dem 1-Schritt Verfahren und dem 2-Schritt Verfahren untersucht. cfu steht für Kolonie-bildende Einheiten (KbE); ISO steht für Durchführung in Anlehnung an ISO: 11290-1:1996 FDAM 1, 1-Schritt steht für Anreicherung der Listerien im 1-Schritt Verfahren mit StrepTag-HisTag-GFP-CBD511_f2 kovalent gekoppelt an Dynabeads M-270 Epoxy. 2-Schritt steht für Anreicherung der Listerien im 2-Schritt Verfahren mit StrepTag-HisTag-GFP-CBD511 f3 und Streptavidin-beschichteten magnetischen Partikeln. Die Werte sind das Mittel aus 2 Versuchen.

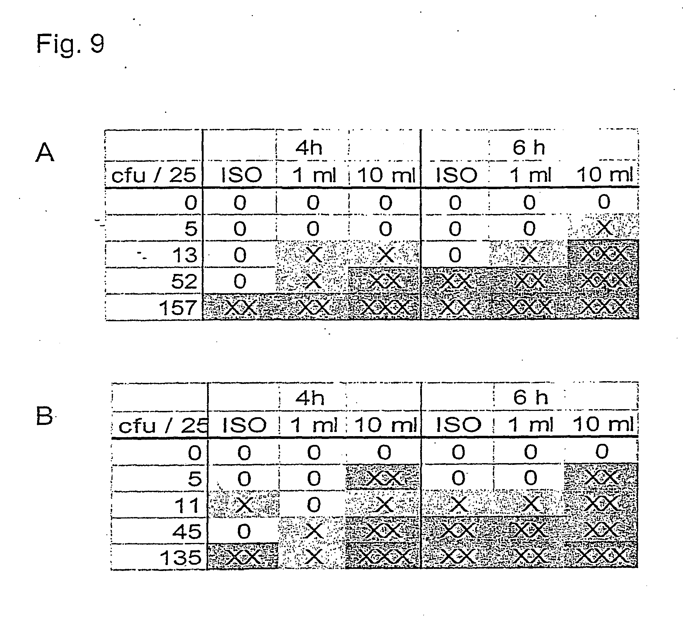

Fig. 9 zeigt die Zeitabhängigkeit des Nachweises von *L. monocytogenes* in rohem Schinken (A) und in Garnelen (B) gemäß ISO-Norm, dem Profos 2-Schritt Verfahren mit 1 ml und dem Profos 2-Schritt Verfahren mit 10 ml. Versuchsdurchführung beschrieben in Experiment 9. 5 Portionen ä 25 g roher Schinken bzw. Garnelen wurden mit 0, 5, 13, 52 und 157 KbE bzw. mit 0, 5, 11, 45 und 135 KbE kontaminiert, in 225 ml Fraser ½ homogenisiert und bei 30 °C inkubiert. Proben wurden zu den angegebenen Zeiten entnommen und gemäß ISO: 11290-1:1996 FDAM1 und nach dem 2-Schritt Verfahren mit 1 ml und mit 10 ml untersucht. cfu steht für Kolonie-bildende Einheiten (KbE), ISO steht für Durchführung in Anlehnung an ISO: 11290-1:1996 FDAM 1, 1 ml steht für Anreicherung der Listerien aus 1ml Nahrungsmittel-Homogenisat im 2-Schritt Verfahren mit StrepTag-HisTag-GFP-CBD511_f3 und Streptavidin-beschichteten magnetischen Partikeln, 10 ml steht für Anreicherung der Listerien aus 10ml Nahrungsmittel-Homogenisat im 2-Schritt Verfahren mit StrepTag-HisTag-GFP-CBD511_f3 und Streptavidin-beschichteten magnetischen Partikeln. Die Werte sind das Mittel aus 2 Versuchen

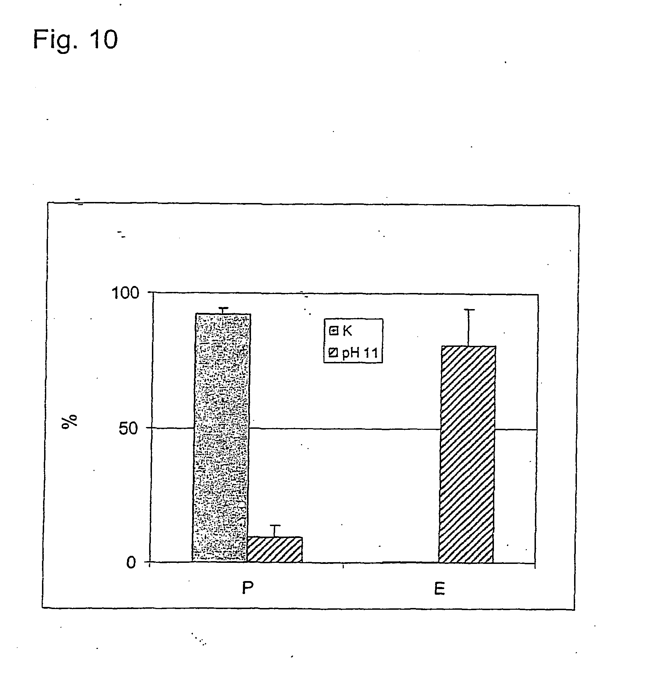

Fig. 10 zeigt die Ablösung der über das 2-Schritt Verfahren an die magnetischen Partikel gebundenen Listerien durch alkalischen Puffer. Dargestellt ist die Menge der an die magnetischen Partikel gebundenen Zellen in Prozent (im Vergleich zu der Zahl) der insgesamt wieder gefundenen Zellen. Versuchsdurchführung beschrieben in Experiment 10. Die Listerien wurden über das 2-Schritt Verfahren an den magnetischen Partikeln immobilisiert. Diese Listerien-Partikel-Komplexe wurden entweder in neutralem Puffer (Kontrolle, K) oder in alkalischem Puffer (pH 11) inkubiert. Nach Abtrennung des Überstandes von den Partikeln wurde beides in seriellen Verdünnungen auf Oxford-Agar plattiert und bei 37 °C inkubiert. "Partikel (P)" bezeichnet die an den magnetischen Partikeln verbliebenen Listerienzellen, "Eluat (E)" bezeichnet die durch den jeweiligen Puffer abgelösten Listerien.

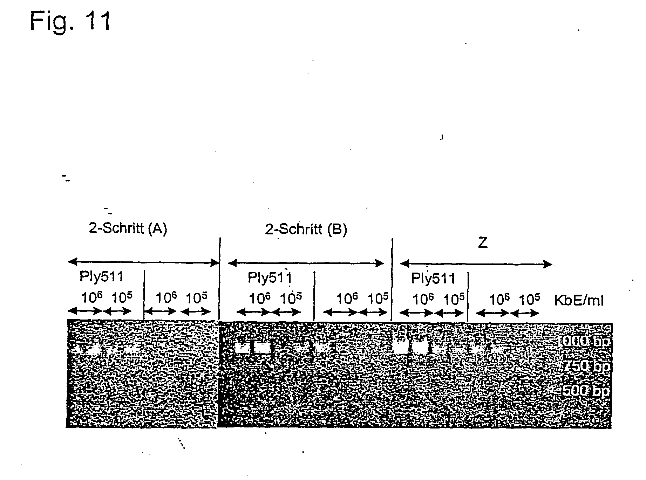

Fig. 11 zeigt die Auftrennung der DNA-Fragmente nach PCR mit genomischer DNA aus *L. monocytogenes* ScottA nach Anreicherung über das 2-Schritt Verfahren. Versuchsdurchführung beschrieben in Experiment 11. Zellen von *L. monocytogenes* wurden über das 2-Schritt-Verfahren, bzw. Zentrifugation aus 1 ml *Listeria* Enrichment Broth acc. FDA (Profos AG) aufkonzentriert. Die an die magnetischen Partikel gebundenen Zellen wurden zum Einen abgelöst von den magnetischen Partikeln und die Zellen mit bzw. ohne Ply511-Behandlung mit Proteinase K und Hitzebehandlung (94 °C) aufgebrochen. Zum Anderen wurden die Zellen an den magnetischen Partikeln mit bzw. ohne Ply511-Behandlung mit Proteinase K und Hitzebehandlung (94 °C) aufgebrochen. Als Kontrolle wurden Zellen nach Zentrifugation mit bzw. ohne Ply511-Behandlung mit Proteinase K und Hitzebehandlung (94 °C) aufgebrochen. 5 µl der Zelllysate wurden in eine PCR eingesetzt und die erhaltenen DNA-Fragmente wurden über Agarosegelelektrophorese aufgetrennt. "2-Schritt" steht für Anreicherung über das 2-Schritt-Verfahren aus 1 ml *Listeria* Enrichment Broth acc. FDA (Profos AG). Spalte (A): zeigt den Aufbruch nach Ablösung der an die magnetischen Partikel gebundenen Zellen mit Natriumphosphat pH 11 und anschließende Zelllyse und Freisetzung der genomischen DNA von *L. monocytogenes* Scott A. Spalte (B) steht für Zelllyse und Freisetzung der genomischen DNA von *L. monocytogenes* Scott A ohne Ablösung von den magnetischen Partikeln. Spalte Z (Zentrifugation) steht für Zelllyse und Freisetzung der genomischen DNA von *L. monocytogenes* Scott A nach Aufkonzentration der Zellen über Zentrifugation. Ply511 steht für Aufbruch der Zellen mit dem Endolysin Ply511. 10⁶ und 10⁵ steht für 1,4 x 10⁶ bzw. 1,4 x 10⁵ KbE / ml im Test.

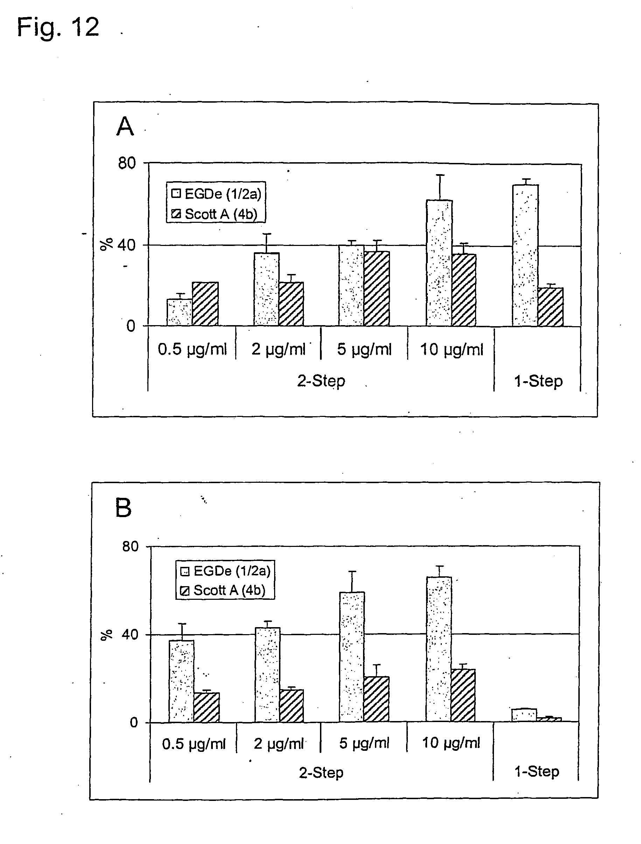

Fig 12 zeigt im 1-Schritt- und 2-Schritt-Verfahren die Konzentrationsabhängigkeit des Nachweises von *L. monocytogenes* (Stamm EGDe und ScottA) aus Frankfurter Würstchen (Fig. 12A) und Mozzarella (Fig. 12B) unter Verwendung des Fusionsproteins Bio-Av-GFP-CBD511_f3. Die Versuchsdurchführung ist in Experiment 12 beschrieben. Dargestellt ist jeweils, wie viel % der insgesamt eingesetzten Listeriazellen der jeweiligen Stämme aus 1ml Proben aus den Lebensmitteln Frankfurter Würstchen (Fig. 12A) und Mozzarella (Fig. 12B) wieder gefunden wurden. Die Werte wurden ermittelt aus jeweils 2 Versuchen.

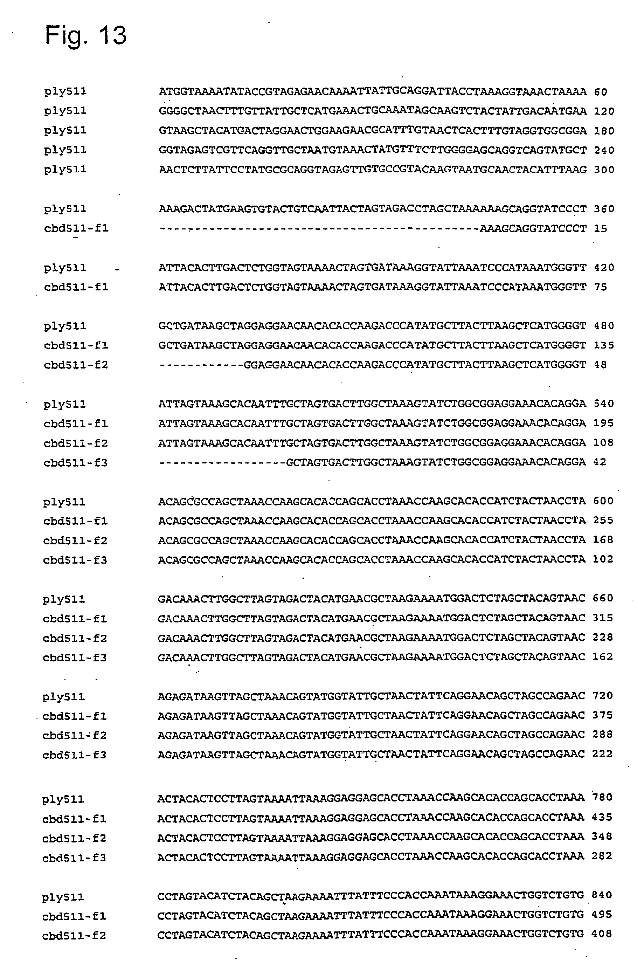

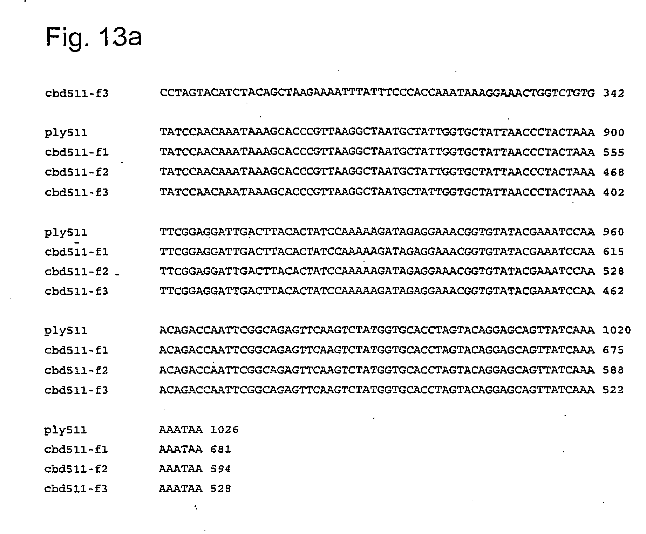

Fig. 13 und 13a zeigen den Vergleich der Nukleinsäuresequenz von Endolysin Ply511 mit den Polypeptidfragmenten CBD511_f1, CBD511_f2, und CBD_f3.

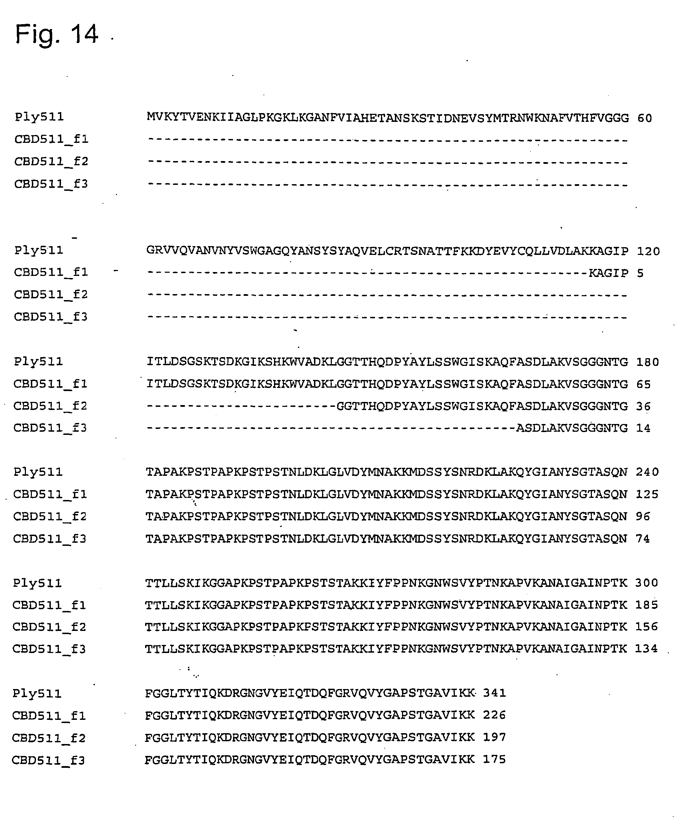

Fig. 14 zeigt den Vergleich der Aminosäuresequenz von Endolysin Ply511 mit den Polypeptidfragmenten CBD511_f1, CBD511_f2, und CBD_f3.

Der Begriff "Listerien" wie hier verwendet bedeutet alle Bakterien, die der Gattung *Listeria* zugeordnet werden. Insbesondere schließt der Begriff Listerien die Species *L. monocytogenes* mit den Serotypen 1/2a, 1/2b, 1/2c, 3a, 3b, 3c, 4a, 4ab, 4b, 4c, 4d, 4e, 7; *L. innocua* mit den Serotypen 3, 6a, 6b, 4ab, U/S; *L. ivanovii* mit dem Serotyp 5; *L*. *seeligeri* mit den Serotypen 1/2a, 1/2b, 1/2c, 4b, 4c, 4d, 6b; *L. welshimeri* mit den Serotypen 1/2a, 4c, 6a, 6b,U/S und *L. grayi* mit dem Serotyp Grayi ein.

Der Begriff "Listerien-Abreicherung" oder "Listerien-Entfernung" wie hier verwendet bedeutet vollständige oder teilweise Entfernung von Listerien aus Probenmaterial.

Der Begriff "Voranreicherung" oder "Anreicherung" wie hier verwendet bezeichnet die Anzucht von Listerien z.B. in einer Lebensmittelprobe die mit entsprechendem Nährmedium versetzt wurde mit dem Ziel die Konzentration/Menge der Listerien in der Probe soweit zu erhöhen, das ein entsprechender Nachweisschritt eine eindeutig positive oder eindeutig negative Aussage ermöglicht.

Der Begriff "Probenmaterial" oder "Probe" wie hier verwendet umfasst sämtliche Lösungen, in denen Listerien nachgewiesen werden sollen oder aus denen Listerien entfernt werden sollen. Beispielhaft für Proben ist die folgende Aufzählung: wässrige Lösungen und Gemische aus Wasser und organischen Lösungsmitteln, Nahrungsmitteln, Medien, Blut, Blutprodukte, Plasma, Serum, Urin, Proteinlösungen, Wasser-Ethanol Gemische. Umfasst sind ferner auch Lösungen, in denen nicht wässrige feste zu untersuchende oder zu isolierende Substanzen gelöst wurden, beispielsweise Protein, DNA, RNA, Zucker, Salze, Nahrungsmittel, Nahrungsmittel-Medien-Homogenisate, Arzneimittel, Impfstoffe, Organische und Anorganische Chemikalien (z.B. NaCl, MgCl₂, Purine, Pyrimidine, usw.).

Der Begriff "Endolysin" wie hier verwendet bezeichnet ein natürlicherweise phagenkodiertes Enzym, das zur Freisetzung neuer Phagen am Ende eines jeweiligen Phagenreproduktionszykluses dient. Diese Endolysine bestehen aus einer enzymatisch aktiven Domäne und einer an die Zellwand der jeweiligen Wirtszelle bindenden Domäne. Zusätzlich sind darunter die ähnlich aufgebauten Autolysine zu verstehen. Diese sind bakteriencodiert und bestehen ebenfalls aus einer enzymatisch aktiven Zellwand hydrolysierenden Domäne und einer an die Zellwand des Zielbakteriums bindenden Domäne.

Der Begriff "CBD" wie hier verwendet bezeichnet Polypeptidfragmente, wobei die jeweilige Aminosäuresequenz einem Bereich in Endolysinen entspricht. Dieser Bereich ist für die Bindung der Endolysine an die Listerien Zellwand verantwortlich. Diese Polypeptidfragmente sind nicht enzymatisch aktiv. Die CBDs können auch als Genfusion mit einem Spacermolekül (GFP, MBP, Biotinylierungsdomänen) mit und ohne einem Affinitäts-Tag (His-Tag, Strep-Tag, Avi-Tag, Biotinylierungsdomänen) vorliegen, oder auch als Genfusion nur mit Affinitäts-Tag (His-Tag, Strep-Tag, Avi-Tag, Biotinylierungsdomänen).

Der Begriff "unspezifische Immobilisierung" oder "ungerichtete Immobilisierung" wie hier verwendet bedeutet, dass die Kopplung einer CBD an eine Matrix über Aminosäurereste (z.B. primäre Amine) erfolgt, die über die gesamte Polypeptidoberfläche verteilt sind. Die Auswahl des für die Kopplung des einzelnen Polypeptidmoleküls verwendeten Restes ist zufällig. Hierbei können die CBDs entweder direkt an aktivierte Gruppen an einer Matrix gebunden werden (z.B. Bindung an Dynabeads M-270 Epoxy, Dynal), oder in die CBD kann chemisch eine Gruppe eingeführt werden (z.B. Einführung von Biotin mit EZ-link-sulfo-NHS-LC-LC-Biotin, Pierce) und über diese die CBD an eine Matrix gebunden werden, die mit einem Liganden für die eingeführte Gruppe beschichtet ist (z.B. Streptavidin).

Der Begriff "gerichtete Immobilisierung" wie hier verwendet bedeutet, dass die Kopplung einer CBD über Aminosäurereste oder andere Reste (z.B. Glykosyslierungen des Proteins) erfolgt, deren Position im Protein (z. B. N- oder C-terminal) bekannt ist. Die Auswahl dieser Gruppen für die Kopplung erfolgt durch die Auswahl geeigneter Reaktionspartner/Linker, die bevorzugt mit diesen Resten reagieren (z.B. Kopplung von Sulfhydrylresten an Iodoacetatreste; Iodoacetat reagiert tausendmal schneller mit Sulfhydrylresten als mit Aminoresten). Der Begriff bedeutet zudem, dass die Nukleotidsequenz der CBD mit der Nukleotidsequenz eines Affinitäts-Tags (z.B. StrepTag oder HisTag) fusioniert ist, der an eine spezifische Matrix bindet (z.B. Streptavidin-beschichtete magnetische Partikel, oder Nickel-Chelat-Liganden). Der Begriff bedeutet zudem, dass die Nukleotidsequenz der CBD mit der Nukleotidsequenz eines Polypeptids fusioniert ist, das von anderen Proteinen erkannt wird in welches diese anderen Proteine ein Molekül an einer definierten Stelle einbauen (z.B. AviTag oder die Biotinylierungsdomäne der Oxalacetatdecarboxylase von *Klebsiella*).

Der Begriff "Oberfläche" oder "Träger" wie hier verwendet umfasst alle Materialien, an die eine Kopplung oder Adhäsion eines CBD-Moleküls möglich ist, wie z.B. Glasoberflächen, Chromatographiematerialien, z.B. Agarose oder Sepharose, Plastikoberflächen, z.B. Polystyrol oder Polypropylen, Filtermaterialien, z.B. Cellulose.

Der Begriff "1-Schritt-Verfahren" wie hier verwendet bezeichnet ein Verfahren, bei dem die CBDs bereits vor der Zugabe der Probe entweder gerichtet oder ungerichtet an einen geeigneten Träger oder eine Oberfläche immobilisiert wurden. Nach Inkubation der immobilisierten CBDs mit der Probe wird der Listerien-CBD-Träger-Komplex von der Probe abgetrennt und evtl. anschliessend gewaschen.

Der Begriff "2-Schritt-Verfahren" wie hier verwendet bezeichnet ein Verfahren, bei dem geeignete nicht immobilisierte CBDs mit der Probe in Kontakt gebracht und inkubiert werden. Die gebildeten Listerien-CBD-Komplexe werden anschliessend mit einem geeigneten Träger oder einer Oberfläche in Kontakt gebracht, so dass die Listerien-CBD-Komplexe über die CBDs an die Träger oder Oberflächen gebunden werden. Nachfolgend werden die Listerien-CBD-Träger-Komplexe von der Probe abgetrennt und evtl. gewaschen. Geeignete CBD sind mit einem Polypeptid oder einer chemischen Gruppe derart modifiziert, dass sie an einen Träger oder eine Oberfläche spezifisch binden, die mit dem jeweiligen Bindungspartner des Polypeptids oder der chemischen Gruppe beschichtet sind.

Die vorliegende Erfindung betrifft Polypeptidfragmente, die aus dem Endolysin Ply511 des Phagen A511 stammen und für die Bindung des Phagen an die Zellwand von Listerien verantwortlich sind. Die Aminosäuresequenz des Volllängen-Endolysins Ply 511 ist in SEQ ID NO:2 und die dafür codierende Nukleinsäuresequenz in SEQ ID NO:1 dargestellt. Überraschenderweise weisen die isolierten erfindungsgemäßen Polypeptidfragmente des Endolysins Ply511 ein Bindungsspektrum abweichend von dem Lysespektrum des Volllängen-Endolysins Ply511 auf. Während das Endolysin Ply511 neben allen Serotypen von Listeria auch mindestens einen Teil der Gattung Bacillus hydrolysiert, werden ausschließlich Listerien von den isolierten erfindungsgemäßen Polypeptidfragmenten erkannt und gebunden.

Die vorliegende Erfindung betrifft Polypeptidfragmente des Endolysins Ply511 mit der Eigenschaft, die Zellwand von Listerien zu binden, wobei die Polypeptidfragmente keine enzymatisch aktiven Zellwand hydrolysierenden Bereiche mehr aufweisen. Die Erfindung betrifft ferner die Nukleinsäuresequenzen, die die erfindungsgemäßen Polypeptidfragmente codieren. Die erfindungsgemäßen Polypeptidfragmente werden im Folgenden auch als "Zellwand bindende Domänen" (CBD) bezeichnet. Die CBD erkennen und binden alle Listerien Serotyp-unabhängig, erkennen und binden darüber hinaus jedoch keine anderen Species.

Die erfindungsgemäßen Polypeptidfragmente weisen eine Aminosäuresequenz (bezogen auf die Volllängen-Sequenz gemäß SEQ ID NO:2) höchstens von Position 116 bis 341 und mindestens von 180 bis 341 auf. Die erfindungsgemäßen Polypeptidfragmente entsprechen also am N-terminalen Ende irgendeiner Position im Bereich von Position 116 bis Position 180 und am C-terminalen Ende der Position 341. Der Bereich von Position 116 bis 180 ist nicht auf genau diesen Bereich festgelegt, sondern kann sich um einige Aminosäurepositionen in Richtung N- oder C-Terminus verschieben, solange die erfindungsgemäßen Polypeptidfragmente keine Zellwand hydrolysierende Aktivität aufweisen und die Zellwand bindende Aktivität erhalten bleibt. Die Erfindung betrifft ferner die Nukleinsäuremoleküle, codierend die beschriebenen bevorzugten Polypeptidfragmente.

Die vorliegende Erfindung betrifft ferner modifizierte Polypeptidfragmente und die Nukleinsäuresequenzen codierend für die modifizierten erfindungsgemäßen Polypeptidfragmente.

Insbesondere können die CBD mit niedermolekularen Substanzen z.B. Biotin verknüpft sein. Dieses kann chemisch in die CBD eingeführt werden oder durch die Fusion der CBD mit einem Polypeptid, in welches in vivo oder in vitro durch ein weiteres Protein Biotin eingeführt wird. Ein solches Polypeptid sind z.B. Biotinylierungsdomänen, d.h. Bereiche in natürlicherweise vorkommenden Polypeptiden die biotinyliert werden. Solche Biotinylierungsdomänen weisen z.B. die Oxalacetatdecarboxylase von Klebsiella (US 5,252,466 und EP 0511747), die Salmonella typhimurium Oxaloacetat Decarboxylase, die Propionibacterium shermanii Transcarboxylase Untereinheit, das Biotin Carboxyl Carrier Protein der Escherichia coli Acetyl-CoA Carboxylase, die Saccharomyces cerevisiae Pyruvat Carboxylase oder die Saccharomyces cerevisiae Acetyl-CoA Carboxylase auf. Ein solches Polypeptid kann aber auch der AviTag (Avidity-Patente US 5,932,433, US 5,874,239 und US 5,723,584) sein. Außerdem kann durch Fusion mit einem Polypeptid, das eine Gruppe trägt, die im Protein nicht oder selten, aber kaum zugänglich vorkommt (z.B. Cystein) anschließend ein Biotin chemisch spezifisch mit dieser Gruppe verknüpft werden. Statt Biotin kann ferner der so genannte Strep-Tag (Skerra, A. & Schmidt, T. G. M. Biomolecular Engineering 16 (1999), 79-86, US 5,506,121) verwendet werden, der eine kurze Aminosäuresequenz ist und an Streptavidin bindet. Ferner kann der His-Tag verwendet werden. Es ist auch möglich, verschiedene Tags miteinander zu kombinieren und so die verschiedenen Bindungsaffinitäten der einzelnen Tags zu nutzen, z.B. Strep-Tag und His-Tag, oder Biotinylierungsdomäne und His-Tag. Die Biotinylierungsdomänen sowie der AviTag, der Strep-Tag sowie der His-Tag werden vorzugsweise über DNA-Rekombinationstechnologie an die CBD gebunden. Bevorzugt besteht das Fusionsprotein aus der Biotinylierungsdomäne der Oxalacetatdecarboxylase von Klebsiella oder dem AviTag, dem Strep-Tag oder dem His-Tag, die an ihrem C-terminalen Ende mit dem N-terminalen Ende der CBD verbunden sind. Eine solche Fusion kann aber auch einer der oben genannten Tags sein, mit dessen C-terminalem Ende der N-Terminus eines weiteren Proteins, das als eine Art "Spacermolekül" verwendet wird, verbunden ist, z.B. GFP oder Maltosebindeprotein Die CBD kann dann über ihr N-terminales Ende an das C-terminale Ende dieses weiteren Proteins gekoppelt sein.

Die Biotinylierungsdomänen können aber auch als "Spacermolekül" (Abstandshalter) wie GFP oder MBP wirken, weil sie größer sind als die anderen erwähnten Tags wie His-Tag, Strep-Tag oder Avi-Tag. Sie können quasi eine Doppelfunktion erfüllen. Da CBDs Fragmente aus größeren Proteinen darstellen, sind sie in der Regel relativ klein (etwa 100 bis 300 Aminosäuren). Deshalb kann es sinnvoll oder notwendig sein, zwischen der CBD-Domäne und der Gruppe, die für die Immobilisierung an den Träger zuständig ist, eine Art Abstandshalter einzuführen. Dies kann zum einen verhindern, dass die CBD durch die Immobilisierung denaturiert wird und damit ihre Bindungsfähigkeit an die Bakterienoberfläche verliert, zum anderen kann die Zugänglichkeit der Bakterien an die CBDs verbessert sowie unspezifische Bindung an die Oberflächen verringert werden, wenn die CBDs weiter von den Oberflächen entfernt sind. Ferner können die Abstandshalter dazu beitragen, dass die Gruppen, die für die Immobilisierung an die Oberflächen verantwortlich ist, besser zugänglich werden, wenn sie nicht direkt an die CBD fusioniert ist.

Die vorstehend beschriebene Kopplung kann gerichtet, z.B. am N- oder C-Terminus oder ungerichtet erfolgen. Die gerichtete Kopplung erfolgt über eine geeignete, reaktive natürlicherweise bei CBD nicht häufig oberflächenexponierte Aminosäure wie Cystein, das an geeigneter Stelle gezielt eingeführt wurde. Vorzugsweise kann auch über andere Aminosäuren direkt, oder wie auch bei Cystein über einen "Spacer" oder "CrossLinker" (Monofunktionell oder bifunktionell) indirekt gekoppelt werden.

Bei der Cysteinkopplung sind alle bifunktionellen Crosslinker mit NH- und SH-reaktiven Gruppen, mit und ohne Zwischenspacer, z.B. 11-Maleimidoundecanoic acid sulfo-NHS oder Succinimidyl-4-[N-maleimidomethyl]-cyclohexane-1-carboxy-[6-amido]caproate möglich. Sofern keine Spacer vorhanden sind, können 8-12 C-Atom-Spacer mit endständiger NH-Gruppe eingefügt werden. Vorzugsweise erfolgt die Cysteinkopplung über eine spezifische Biotinylierung des Cysteins durch z.B. EZ-Link-PEO-Maleimide activated Biotin (Pierce).

Die erfindungsgemäßen Polypeptidfragmente können für die nachstehend beschriebenen Verfahren zur Anreicherung, zur Entfernung und zum Nachweis von Listerien verwendet werden.

Die vorliegende Erfindung betrifft ein Verfahren zur Anreicherung von Listerien aus einer Probe (so genanntes 1-Schritt-Verfahren), umfassend die Schritte:
a) Inkubation oder in Kontakt bringen einer Probe mit einer CBD, die unspezifisch oder gerichtet, an einem festen Träger immobilisiert ist
b) Trennen des Träger-CBD-Listerien-Komplexes von der Probe, und
c) gegebenenfalls Abwaschen unspezifisch am Träger-CBD-Listerien-Komplex haftender Bestandteile der Probe.

Für das erfindungsgemäße Anreicherungsverfahren ist die CBD, also die erfindungsgemäßen Polypeptide, an feste Träger gekoppelt. Die festen Träger können magnetische oder nicht magnetische Partikel sowie Füllmaterialien für Chromatographiesäulen (z.B. Sepharosematerialien), Cellulose, Filtrationsmedien, Glaspartikel, Zentrifugations- oder Sedimentationsmaterialien (z.B. Agarosepartikel) sein.

Die Kopplung der erfindungsgemäßen Polypeptide, nachstehend auch CBD genannt, kann unspezifisch, oder aber bevorzugt gerichtet, über z.B. eine selektive Biotinylierung, oder gekoppelt über einen Spacer oder Linker erfolgen. Die CBD kann unspezifisch an chemisch aktivierte Festphasen z.B. Festphasen mit Epoxygruppen, Tosyl- oder NHS-Gruppen über Reaktion mit Aminosäureseitenketten der CBD gebunden werden. Die Zugänglichkeit der CBD für Listerien kann z.B. dadurch erhöht werden, dass die CBD in einer Proteinfusion mit einer Polypeptidkette vorliegt, die eine höhere Affinität zu bestimmten aktivierten Oberflächen als die CBD (z.B. GFP an aktiviertes Polystyrol) aufweist. Insbesondere kann das erfindungsgemäße Polypeptid die vorstehend beschriebenen Eigenschaften und Modifizierungen aufweisen.

Die Anreicherung der Listerien kann in einem Verfahren auf magnetischer Basis oder über chromatographische Verfahren oder dem so genannten "Batch"-Verfahren erfolgen.

Die Dauer der Inkubation der Probe mit dem entsprechenden mit CBD gekoppelten Trägermaterial muss an die jeweilige Probe angepasst werden und kann zwischen 1 min und 24 h variieren, insbesondere für etwa 5-60 min oder etwa 30-180 min oder bei Bedarf auch über Nacht.

Besteht das mit der CBD beschichtete Trägermaterial aus magnetischen Partikeln, wird die Probe mit dem Trägermaterial inkubiert. Anschließend wird der Träger-CBD-Listerien-Komplex durch Anlegen eines Magnetfeldes magnetisch von der Probe separiert. Beim Batch-Verfahren wird die mit Listerien belastete Probe mit dem Trägermaterial, an das die erfindungsgemäße CBD kovalent gekoppelt sind, vermischt und gemeinsam inkubiert. Anschließend kann der Träger-CBD-Listerien-Komplex von der Probe abzentrifugiert oder absedimentiert werden oder in eine Säule gefüllt und eluiert oder abfiltriert werden. Die Anreicherung über das Batch-Verfahren, d.h. mit Vorinkubation von Probe und mit den entsprechenden CBD gekoppelten Trägermaterialien, kann insbesondere bei sehr niedrigen Listerienkonzentrationen sinnvoll sein.

Die Anreicherung von Listerien über Chromatographiesäulen kann aber auch im reinen Durchflussverfahren erfolgen. Dazu wird zunächst das mit der CBD beladene Trägermaterial auf eine Chromatographiesäule gegossen. Die mit Listerien belastete Probe wird auf diese Säule aufgetragen und fließt durch diese, wobei die Listerien an die CBD binden und auf der Säule bleiben. Die Probe selbst zeigt idealerweise keine Wechselwirkung mit dem Chromatographiematerial und befindet sich im Durchlauf. Die Flussrate ist abhängig von Volumen und Geometrie der Säule. Die Flussrate ist ferner abhängig von Volumen und Listeriengehalt der Probe, um durch eine möglichst lange Kontaktzeit zwischen Säule und Listerien auch bei niedrigen Listerienkonzentrationen eine effiziente Abreicherung zu erzielen. Die Kontaktzeit ist dabei die Zeit, die die Probe vom Auftragen auf die Säule bis zum Herausfließen benötigt. Das auf der Säule gebundene Listerien kann durch Waschen mit geeigneten Puffern wieder von der Säule entfernt werden, so dass die Säulen mehrfach benutzt werden können.

Die vorliegende Erfindung betrifft ferner ein Verfahren zur Anreicherung von Listerien aus einer Probe (so genanntes 2-Schritt-Verfahren), umfassend die Schritte:
a) Inkubation oder in Kontakt bringen einer Probe mit CBD, die mit einem Polypeptid fusioniert oder mit einer chemischen Gruppe modifiziert ist, so dass sie spezifisch an einen Träger bindet, die mit dem jeweiligen Bindepartner des Polypeptids oder der chemischen Gruppe beschichtet ist
b) in Kontakt bringen und Inkubation von Listerien-CBD-Komplex mit einem Träger die mit dem jeweiligen Bindepartner des Polypeptids oder der chemischen Gruppe beschichtet ist
c) Trennen des Träger-CBD-Listerien-Komplexes von der Probe, und
d) gegebenenfalls Abwaschen unspezifisch am Träger-CBD-Listerien-Komplex haftender Bestandteile der Probe.

Insbesondere kann das erfindungsgemäße Polypeptid, nachstehend auch CBD genannt, die vorstehend beschriebenen Eigenschaften und Modifizierungen aufweisen.

Die Kopplung der CBD an ein weiteres Protein, über das Biotin eingeführt wird, kann gerichtet, z.B. am N- oder C-Terminus oder ungerichtet erfolgen. Die gerichtete Kopplung erfolgt über eine geeignete, reaktive natürlicherweise bei CBD nicht häufig oberflächenexponierte Aminosäure wie Cystein, das an geeigneter Stelle gezielt eingeführt wurde. Vorzugsweise kann auch über andere Aminosäuren direkt, oder wie auch bei Cystein über einen "Spacer" oder "CrossLinker" (Monofunktionell oder bifunktionell) indirekt gekoppelt werden.

Bei der Cysteinkopplung sind alle bifunktionellen Crosslinker mit NH- und SH-reaktiven Gruppen, mit und ohne Zwischenspacer, z.B. 11-Maleimidoundecanoic acid sulfo-NHS oder Succinimidyl-4-[N-maleimidomethyl]-cyclohexane-1-carboxy-[6-amido]caproate möglich. Sofern keine Spacer vorhanden sind, können 8-12 C-Atom-Spacer mit endständiger NH-Gruppe eingefügt werden. Vorzugsweise erfolgt die Cysteinkopplung über eine spezifische Biotinylierung des Cysteins durch z.B. EZ-Link-PEO-Maleimide activated Biotin (Pierce). Ferner kann die Kopplung über bekannte Kopplungsreaktionen an Proteinreste, wie z.B. Carboxyl-, Amino-, Hydroxyl- oder Sulfhydrylreste erfolgen.

Die Dauer der Inkubation und die Abtrennung des Träger-CBD-Listerien-Komplexes erfolgt wie für das vorstehende 1-Schritt-Verfahren.

Die erfindungsgemäßen Verfahren sind nicht nur zur Anreicherung sondern auch zur Entfernung von Listerien aus einer Probe geeignet.

Die vorliegende Erfindung betrifft ferner ein Verfahren zum Nachweis von Listerien in einer Probe. Der Listerien-Nachweis umfasst weitere Schritte im Anschluss an die oben beschriebenen Verfahrensschritte zur Anreicherung, wobei die nachstehenden Nachweise sowohl mit dem 1-Schritt-Verfahren als auch mit dem 2-Schritt-Verfahren durchgeführt werden können.

Beispielsweise können folgende Nachweise verwendet werden: Nachweis der Listerien im Träger-CBD-Listerien-Komplex oder nach Ablösung vom Trägermaterial, mittels selektiven Wachstumsbedingungen z.B. Ausplattieren und Inkubieren auf Selektivmedienplatten, Nachweis der Zielzellen-DNA z.B. PCR oder DNA-Hybridierungsmethoden, Nachweis der Ziel-Zellen-Zellwand oder Bestandteilen davon z.B. über Zellbindedomänen von Endolysinen oder Antikörpern, Nachweis von Ziel-Zellen-Bestandteilen, z.B. Proteinen über ELISA oder Enzymen über ihre Aktivität, oder von z.B. ATP, Nachweis über einen Ziel-Zellen-spezifischen Bakteriophagen z.B. A511-luxA. Vorzugsweise wird der Nachweis der Zielzellen-DNA z.B. mittels PCR oder der Nachweis von Ziel-Zellen-Bestandteilen, z.B. von Proteinen mittels ELISA nach Aufbruch der Listerien durch ein Listerien-spezifisches Endolysin z.B. Ply511 durchgeführt.

Ferner kann der Nachweis der Listerien im Träger-CBD-Listerien-Komplex oder nach Ablösung vom Trägermaterial mittels einer weiteren CBD erfolgen, die mit einem enzymatisch aktiven Polypeptid gekoppelt ist, so dass über die enzymatische Reaktion das Reaktionsprodukt nachgewiesen werden kann. Ferner kann die zusätzliche CBD mit einem fluoreszierenden Protein und mit einem Tag fusioniert sein (z.B. StrepTag-HisTag-GFP-CBD) sein an den anschließend ein Konjugat aus Enzym und einem Protein bindet, das an den Tag spezifisch bindet (z.B. Streptavidin-alkalische Phosphatase Konjugat).

Die erfindungsgemäßen Verfahren zeichnen sich durch die folgenden Vorteile aus:
● Es ist nur ein einziges Bindemolekül, nämlich die erfindungsgemäße CBD, notwendig, um alle Listerien Serotyp-unabhängig zu erkennen und zu binden.
● Man benötigt wesentlich weniger Menge an CBD pro Verfahren. Da bei einer zu untersuchenden Probe nicht bekannt ist, welche Listerien welcher Serovargruppe in der Probe vorliegen, muss mit den Bindemolekülen des Stands der Technik, etwa mit CBD118 oder mit CBD500, mengenmäßig eine wesentlich größere Menge eingesetzt werden, um ausreichend sensitiv gegenüber allen Serovargruppen zu sein. Dagegen sind die erfindungsgemäßen CBDs - die alle Serovar-Typen von Listeria binden - bei geringerer Menge an eingesetztem Polypeptid gleich sensitiv.
● Man benötigt daher auch weniger Trägermaterial, z.B. magnetische Partikel. Träger sind immer eine Quelle möglicher Störfaktoren z.B. für nachfolgende Nachweisverfahren. So können über Bakterien oder andere Probenbestandteile, die unspezifisch an den jeweiligen Träger binden, falsche Ergebnisse erzielt werden. Weiterhin ist der Träger ein entscheidender Kostenfaktor.
● Bei gleicher Inkubationszeit ermöglicht das vorliegende Verfahren eine schnellere Anreicherung der Zielkeime, so dass sich eine Zeitersparnis ergibt.
● Beim sogenannten 2-Schritt-Verfahren können Bindemoleküle gegen verschiedene Keime gleichzeitig eingesetzt werden, z.B. gegen Listeria und Salmonella, und mit demselben Träger (z. B. Streptavidin beschichtete magnetische Partikel) aus der Probe angereichert werden. Anschließend können dann über einen nachfolgenden spezifischen Nachweis die einzelnen Species und/oder Serotypen bestimmt werden.

Die vorliegende Erfindung betrifft ferner einen Kit, enthaltend einen Träger immobilisiert mit den erfindungsgemäßen Polypeptidfragmenten sowie die für die Anreichung und den Nachweis der Listerien erforderlichen Pufferlösungen, z.B. Waschpuffer, Ablösepuffer und/oder Zellaufbruchspuffer. Die vorliegende Erfindung betrifft ferner einen Kit, enthaltend ein modifiziertes erfindungsgemäßes Polypeptidfragment, einen Träger, immobilisiert mit dem entsprechenden- Bindepartner der Modifikation des Polypeptidfragments sowie die für die Anreichung und den Nachweis der Listerien erforderlichen Pufferlösungen, z.B. Waschpuffer, Ablösepuffer und/oder Zellaufbruchspuffer.

Die folgenden Beispiele erläutern die Erfindung und sind nicht als einschränkend aufzufassen. Sofern nicht anders angegeben, wurden molekularbiologische Standardmethoden verwendet, wie z.B. von Sambrook et al., 1989, Molecular cloning: A Laboratory Manual 2. Auflage, Cold Spring Harbor Laboratory Press, Cold Spring Harbor, New York, beschrieben.

### Experiment 1: Nachweis der Bindung der verschieden langen CBD511 Fusionen an Listeria

Die verschiedenen CBD511 Fusionsproteine StrepTag-HisTag-GFP-CBD511_f1, StrepTag-HisTag-GFP-CBD511_f2 und StrepTag-HisTag-GFP-CBD511 f3 -heterolog exprimiert in E. coli HMS174(DE3) - wurden über Ni-Affinitätschromatographie (Qiagen) nach Angaben des Herstellers des Säulenchromatographiematerials aufgereinigt. 50 µl einer ÜN-Kultur von Listeria monocytogenes ScottA (serovar 4b) oder Listeria monocytogenes ProCC 679 (serovar 1/2a) wurde mit ca. 2 µg des gereinigten Proteins vermischt und für 2 min bei RT inkubiert. Nach Zugabe von 1 ml PBST (10 mM Natriumphosphat pH 8, 150 mM NaCl, 0.05 % Tween 20) wurden die Zellen abzentrifugiert, 2 x in 0.5 ml gewaschen und in 50 ml PBST resuspendiert. Die Bindung wurde unter dem Fluoreszenzmikroskop kontrolliert. Es hat sich gezeigt, dass die HisTag-GFP-CBD-Varianten beide Listeria Serotypen 4b und 1/2a binden.

### Experiment 2: Gattungsspezifische Bindung des StrepTag-GFP-CBD511

Die Listerien wurden über Nacht in 2 ml Listeria Enrichment Broth acc. FDA (Profos AG) bei 30 oder 37 °C gezogen, Bacillus, Chryseobcterium, Citrobacter, Escherichia, Enterobacter, Klebsiella, Kocuria, Pseudomonas, Salmonella, Stenotrophomonas und Yersinia in Lauria-Bertani Bouillon (Profos AG) bei 30 oder 37 °C. Campylobacter wurde in Campylobacter Anreicherungsbouillon (Profos AG) unter semiaerober Atmosphäre (CampyGen-Oxoid) bei 42 °C gezogen, Clostridium in TYG-Medium bei 42 °C unter anaerober (Genbox anaer, Biomerieux) Atmosphäre. Lactobacillus wurde bei 37 °C in MRS-Bouillon (Profos AG) kultiviert, Staphylococcus in BHI-Bouillon (Profos AG). Die Zellen wurden abzentrifugiert 1 x gewaschen in 1 ml TBST (10 mM TrisHCl, pH 8, 150 mM NaCl, 0.05 % Tween 20), in 200 µl PBST resuspendiert und für 15 min bei 80 °C hitzeinaktiviert.

### a) NC-Test

### Untersuchung der Bindung der Fusionsproteine an immobilisierte Listeria Zellen:

Etwa 20-30 µl dieser Suspensionen wurden auf eine Nitrocellulosemembran (Sartorius AG) aufgetragen und 30 min bei 80 °C gebacken. Nach Befeuchtung mit TBST wurde die Membran für 30 min in BSA-Lösung (1 % w/v in TBST) blockiert und mit StrepTag-HisTag-GFP-CBD511-f2, StrepTag-HisTag-GFP-CBD500 oder StrepTag-HisTag-GFP-CBD118 (jeweils 10 µg/ml) bei RT unter leichtem Schütteln 30 min inkubiert. Anschließend wurde die Membran 2x 15 min in TBST gewaschen und für 30 min mit Streptaktin-AP-Konjugat (IBA) in TBST inkubiert. Nach dem Waschen (4 x 15 min TBST) wurde die Membran in Färbelösung (100 mM TrisHCl pH 9.5, 100 mM NaCl, 5 mM MgCl2, 0.18 mg / ml NBT (Applichem) und 0.22 mg / ml BCIP (Applichem)) inkubiert für etwa 15-30 min inkubiert. Die Reaktionslösung wurde abgezogen, die Membran in Wasser gewaschen und getrocknet.

**Tabelle 1: zeigt das Ergebnis der Bindung der Zellbindedomäne CBD511 an Nicht-Listerien.**

| **Spezies** | **Stamm** Profos Culture Collection (ProCC) | alternative Stammbezeichnung | **Bindung von CBD511** |
|---|---|---|---|
| *Bacillus cereus* | 332 | | nein |
| *Bacillus mycoides* | 328 | | nein |
| *Bacillus sp*. | 534 | | nein |
| *Bacillus thuringiensis* | 19 | | nein |
| *Bacillus thuringiensis* | 471 | CC5 | nein |
| *Bacillus vallismortis* | 20 | | nein |
| *Bacillus subtilis* | 310 | | nein |
| | | | |
| *Campylobacter lari* | 986 | | nein |
| *Campylobacter jejuni* | 851 | NC12662-02 | nein |
| | | | |
| *Clostridium perfringens* | 780 | NCTC 3110 | nein |
| *Clostridium perfringens* | 1029 | | nein |
| *Clostridium perfringens* | 1030 | | nein |
| *Clostridium sordelli* | 1039 | | nein |
| | | | |
| *Chryseobacterium meningosepticum* | 333 | | nein |
| *Chryseobacterium sp.* | 337 | | nein |
| | | | |
| *Citrobacter amalonaticus* | 367 | DSMZ 4593 | nein |
| *Citrobacter freundii* | 352 | | nein |
| *Citrobacter freundii* | 249 | | nein |
| | | | |
| *Enterobacter aerogenes* | 205 | DSMZ 30053 | nein |
| *Enterobacter amnigenus* | 473 | BB5 | nein |
| *Enterobacter cloacae* | 18 | | nein |
| *Enterobacter asburiae* | 16 | | nein |
| | | | |
| *Enterococcus durans* | 879 | | nein |
| | | | |
| *Escherichia coli* | 687 | ECOR 05 | nein |
| *Escherichia coli* | 734 | ECOR 52 | nein |
| | | | |
| *Klebsiella granulomatis* | 75 | KC2668 | nein |
| *Klebsiella oxytoca* | 366 | DSMZ 5175 | nein |
| *Klebsiella pneumoniae* | 207 | DSMZ 30102 | nein |
| | | | |
| *Kocuria rhizophila* | 22 | | nein |
| | | | |
| *Lactobacillus casei (393)* | 356 | 393 | nein |
| *Lactobacillus casei (862)* | 351 | 862 | nein |
| *Lactobacillus casei (864)* | 349 | 864 | nein |
| *Lactobacillus plant (2410)* | 350 | 2410 | nein |
| | | | |
| *Micrococcus luteus* | 24 | | nein |
| | | | |
| *Pseudomonas fluoreszens* | 370 | | nein |
| | | | |
| *Ralstonia picketti* | 420 | | nein |
| | | | |
| *Salmonella enteritits SZ 15* | 478 | | nein |
| *Salmonella dublin* | 959 | SL 5608 20031113 | nein |
| *Salmonella berta* | 956 | IS 69 20031113 | nein |
| | | | |
| *Staphylococcus aureus* | 456 | | nein |
| *Staphylococcus aureus* | 457 | | nein |
| *Staphylococcus aureus* | 458 | | nein |

### b) LB-Test

### Untersuchung der Bindung von Listerien an magnetische Partikel über die Fusionsproteine:

0.5 ml einer frischen ÜN-Kultur wurden auf 2 ml Listeria Enrichment Broth acc. FDA (Profos AG) gegeben und bis zu einer OD600 von ca. 1 bei 37°C gezogen (entspricht 0.5 bis 1 x 10⁹ KbE/ml). Die Kulturen wurden in PBST auf ca. 1 x 10⁴ KbE / ml verdünnt. Zu jeweils 1 ml der Zellverdünnungen wurden 1 µg StrepTag-IIisTag-GFP-CBD511-f3 Fusionsproteins gegeben und kurz gemischt. Nach Zugabe von 50 µg MagPrep-Streptavidin (Merck) wurden die Ansätze 20 min im Über-Kopf-Rolator bei RT inkubiert. Anschließend wurden die Komplexe aus magnetischen Partikeln, Fusionsprotein und Listerien im Magnetfeld an der Gefäßwandung gesammelt und der Überstand (ÜS1) in ein steriles Gefäß gegeben. Der Partikel-Protein-Listerien-Komplex wurde in 1 ml PBST 10 min im Über-Kopf-Rolator gewaschen, im Magnetfeld an der Gefäßwandung gesammelt und der Überstand zu ÜS1 hinzugefügt. Der Komplex wurde in 1 ml PBST resuspendiert. Anschließend wurden serielle Verdünnungen in PBST der vereinten Überstände und der resuspendierten Komplexe auf Oxford-Agar (Profos AG) ausplattiert und für 24 h bei 37 °C inkubiert. Die Platten wurden ausgezählt und der Anteil der an den magnetischen Partikeln haftenden Listerien in Prozent der insgesamt wieder gefundenen Listerien berechnet.

Tabelle 2: zeigt das Ergebnis der Serotypabhängigkeit der Bindeeigenschaften verschiedener Zellbindedomänen von Listerien-Endolysinen an Stämme der Gattung Listeria. CBD118 steht für die Zellbindedomäne des Endolysins Ply118; CBD500 steht für die Zellbindedomäne des Endolysins Ply500; CBD511_f2 und CBD511_f3 stehen für die Varianten der Zellbindedomäne des Endolysins Ply511; NC-Test bedeutet: die Bindung der jeweiligen StrepTag-HisTag-GFP-CBD. Fusionen wurde ermittelt über die Bindung an auf Nitrocellulosemembran immobilisierte Zellen (Experiment 2a) und Nachweis über Streptaktin-Alkalische-Phosphatase-Konjugat; LB-Test bedeutet: die Bindung wurde ermittelt über die Entfernung der Listerien aus Lösungen mittels Streptavidin-beschichteter magnetischer Partikel und der monobiotinylierten Zellbindedomäne Bio-GFP-CBD5 11_f3 (Experiment 2b).

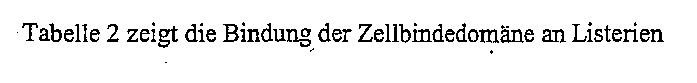

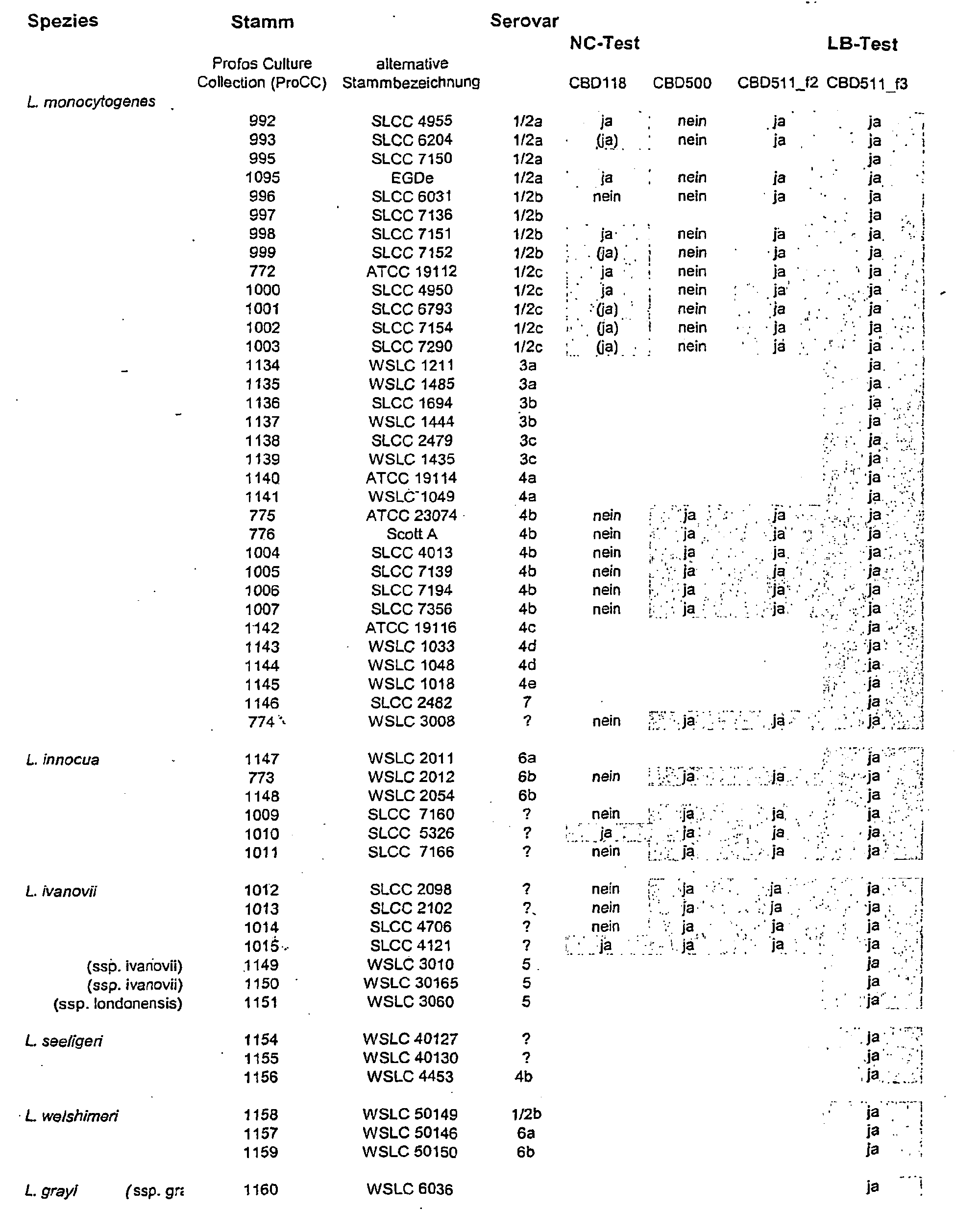

### Experiment 3: Vergleich der Zellbindeeigenschaft des StrepTag-HisTag-CBD511-f3 mit StrepTag-HisTag-CBD500

0.5 ml einer frischen ÜN-Kultur (L. monocytogenes ScottA, serovar 4b; L. moncytogenes EGDe, serovar 1/2a) wurden auf 2 ml Listeria Enrichment Broth acc. FDA (Profos AG) gegeben und bis zu einer OD600 von ca. 1 bei 37 °C gezogen (entspricht 0.5 bis 1 x 10⁹ KbE /ml). Die Kulturen wurden in PBST auf ca. 1 x 10⁴ KbE / ml verdünnt. Zu jeweils 1 ml der Zellverdünnungen wurden 1 µg, 5 µg und 10 µg des StrepTag-HisTag-GFP-CBD511-f3 Fusionsproteins bzw. StrepTag-I-IisTag-CBD500 Fusionsproteins gegeben und kurz gemischt. Nach Zugabe von 50 µg MagPrep-Streptavidin (Merck) wurden die Ansätze 20 min im Über-Kopf-Rolator bei RT inkubiert. Anschließend wurden die Komplexe aus magnetischen Partikeln, Fusionsprotein und Listerien im Magnetfeld an der Gefäßwandung gesammelt und der Überstand (ÜS1) in ein steriles Gefäß gegeben. Der Partikel-Protein-Listerien-Komplex wurde in 1 ml PBST 10 min im Über-Kopf-Rolator gewaschen, im Magnetfeld an der Gefäßwandung gesammelt und der Überstand zu ÜS1 hinzugefügt. Der Komplex wurde in 1 ml PBST resuspendiert. Anschließend wurden serielle Verdünnungen in PBST der vereinten Überstände und der resuspendierten Komplexe auf Oxford-Agar (Profos AG) ausplattiert und für 24 h bei 37 °C inkubiert. Die Platten wurden ausgezählt und der Anteil der an den magnetischen Partikeln haftenden Listerien in Prozent der eingesetzten Zellen berechnet.

Im Gegensatz zur CBD500 konnten mit der CBD511 auch Listerien des Serotyps 1/2a proteinkonzentrationsabhängig angereichert werden. Der Serotyp 4b wird sowohl mit der CBD511 als auch der CBD500 proteinkonzentrationsabhängig angereichert.

### Experiment 4: Abhängigkeit der Bindung von Listeria an magnetische Partikel im 2-Schritt-Verfahren von der Konzentration an biotinylierten CBD511 Konstrukten - Bio-OD-CBD511_f3 und Bio-Av-GFP-CBD511_f3

N-terminal an die CBD511_f3 wurde mit molekularbiologischen Standardtechniken entweder die Biotinylierungsdomäne der α-Untereinheit der *Klebsiella pneumoniae* Oxalacetat Decarboxylase (GenBank Accession Nr. J03885, Patente US 5,252,466, EP 0511747, Schwarz et al., Journal of Biological Chemistry 263 (1988), 9640-9645) als Biotinylierungsdomäne oder der Avi-Tag (US 5,723,584; US 5,874239) kloniert. Wegen der geringen Größe des Avi-Tags wurde zusätzlich GFP als "Linkerdomäne" eingeführt, wie in Abbildung 1A dargestellt. Die Konstrukte wurden Bio-OD-CBD511_f3 bzw. Bio-Av-GFP-CBD511_f3 genannt. Die Bindetests mit Bio-OD-CBD511_f3 (Fig. 4A) und Bio-Av-GFP-CBD511_f3 (Fig. 4B) wurden folgendermaßen durchgerührt. Jeweils 0.5 ml einer frischen ÜN-Kultur (*L. monocytogenes* ScottA, serovar 4b) wurde auf 2 ml *Listeria* Enrichment Broth acc. FDA (Profos AG) gegeben und bis zu einer OD₆₀₀ von ca. 1 bei 37 °C gezogen (entspricht 0.5 bis 1 x 10⁹ KbE /ml). Die Kulturen wurden in einem PBST (10 mM Natriumphosphat pH 8, 150 mM Natriumchlorid, 0.05 % Tween 20) auf 1 x 10³ - 1 x 10⁴ KbE/ml eingestellt und weitere serielle Verdünnungen zur Ermittlung der tatsächlichen Zellzahl auf Oxford-Agar (Profos AG) ausplattiert. Zu 1 ml Zellverdünnung wurde Bio-OD-CBD511_f3 in den Konzentrationen 0, 0.001, 0.005, 0.01, 0,02, 0.1, 0.3, 0.5, 0.75, 1, 1.5, 2, 3, 4, 5, 10, 20 und 40 µg/ml zugegeben, Bio-Av-GFP-CBDS 11_f3 in den Konzentrationen 0.5, 1, 2 und 10 µg/ml, kurz geschüttelt und nach Zugabe von Streptavidin-Magnetic-Particles (Roche) zu 50 µg/ml wurden die Ansätze für 20 min im Über-Kopf-Rolator bei RT inkubuiert. Anschließend wurden die Partikel-Listerien-Komplexe im Magnetfeld an der Gefäßwandung gesammelt, der Überstand abgezogen und in ein steriles 2 ml Eppendorfcup gegeben. Der Partikel-Listerien-Komplex wurde in 1 ml PBST 10 min im Über-Kopf-Rolator gewaschen, im Magnetfeld an⁻ der Gefäßwandung gesammelt, der Überstand zum ersten Überstand hinzugegeben.

Der Listerien-Partikel-Komplex wurde in 1 ml PBST resuspendiert. Anschließend wurden serielle Verdünnungen der vereinten Überstände und der resuspendierten Komplexe in PBST auf Oxford-Agar (Profos AG) ausplattiert und für 24 h bei 37 °C inkubiert. Die Platten wurden ausgezählt und der Anteil der an den magnetischen Partikeln haftenden Listerien in Prozent der eingesetzten Zellen berechnet.

Es hat sich gezeigt dass mit Bio-OD-CBD_f3 (A) ab einer Konzentration von 0,3 µg/ml die maximale Ausbeute an Listeria im 2-Schritt-Verfahren erreicht wird und über einen weiten Konzentrationsbereich bis 40 µg/ml konstant bleibt, bei Bio-Av-GFP-CBD_f3 (B) wird ab etwa 2 µg/ml Protein die maximale Ausbeute an gebundenen Listerien erreicht..

### Experiment 5: Abhängigkeit der Zellbindung des 2-Schritt-Verfahrens vom pH

0.5 ml einer frischen ÜN-Kultur (*L. monocytogenes* ScottA, serovar 4b; *L. moncytogenes* EGDe, serovar 1/2a) wurden auf 2 ml *Listeria* Enrichment Broth acc. FDA (Profos AG) gegeben und bis zu einer OD₆₀₀ von ca. 1 bei 37 °C gezogen (entspricht 0.5 bis 1 x 10⁹ KbE /ml). Die Kulturen wurden in einem PufferX (10 mM Natriumcitrat, 10 mM Natriumphosphat), eingestellt auf den jeweiligen pH-Wert, auf ca. 1 x 10⁴ KbE / ml verdünnt. Zu jeweils 1 ml der Zellverdünnungen wurden 5 µg des Bio-Av-GFP-CBD_f3 Fusionsproteins gegeben und kurz gemischt. Nach Zugabe von 50 µg MagPrep-Streptavidin (Merck) wurden die Ansätze 20 min im Über-Kopf-Rolator bei RT inkubiert. Anschließend wurden die Komplexe aus magnetischen Partikeln, Fusionsprotein und Listerien im Magnetfeld an der Gefäßwandung gesammelt und der Überstand (ÜS1) in ein steriles Gefäß mit einem 1/10 Volumen 10 x PBST gegeben. Der Partikel-Protein-Listerien-Komplex wurde in 1 ml Puffers X 10 min im Über-Kopf-Rolator gewaschen, im Magnetfeld an der Gefäßwandung gesammelt und der Überstand zu ÜS1 hinzugefügt. Der Komplex wurde in 1 ml PBST resuspendiert. Anschließend wurden serielle Verdünnungen der vereinten Überstände und der resuspendierten Komplexe in PBST auf Oxford-Agar (Profos AG) ausplattiert und für 24 h bei 37 °C inkubiert. Die Platten wurden ausgezählt und der Anteil der an den magnetischen Partikeln haftenden Listerien in Prozent der eingesetzten Zellen berechnet. Es hat sich gezeigt, dass das 2-Schritt-Verfahren zur Anreicherung von Listerien des Serotyps 4b und des Serotyps 1/2a über den pH-Bereich von 5 bis 10 gleich bleibende Ausbeuten ergibt.

### Experiment 6: Abhängigkeit der Zellbindung des 2-Schritt-Verfahrens vom Salzgehalt

0.5 ml einer frischen ÜN-Kultur (L. monocytogenes ScottA, serovar 4b; L. moncytogenes EGDe, serovar 1/2a) wurden auf 2 ml Listeria Enrichment Broth acc. FDA (Profos AG) gegeben und bis zu einer OD₆₀₀ von ca. 1 bei 37 °C gezogen (entspricht 0.5 bis 1 x 10⁹ KbE /ml). Die Kulturen wurden in 10 mM Natriumphosphat, pH 8, 0.05 % Tween20 und einem Salzgehalt von 0, 100, 200, 400, 600, 800 und 1000 mM NaCl auf ca. 1 x 10⁴ KbE / ml verdünnt. Zu jeweils 1 ml der Zellverdünnungen wurden 5 µg des StrepTag-HisTag-GFP-CBD511-f3 Fusionsproteins gegeben und kurz gemischt. Nach Zugabe von 50 µg MagPrep-Streptavidin (Merck) wurden die Ansätze 20 min im Über-Kopf-Rolator bei RT inkubiert. Anschließend wurden die Komplexe aus magnetischen Partikeln, Fusionsprotein und Listerien im Magnetfeld an der Gefäßwandung gesammelt und der Überstand (ÜS1) in ein steriles Gefäß gegeben. Der Partikel-Protein-Listerien-Komplex wurde in 1 ml PBST 10 min im Über-Kopf-Rolator gewaschen, im Magnetfeld an der Gefäßwandung gesammelt und der Überstand zu ÜS1 hinzugefügt. Der Komplex wurde in 1 ml PBST resuspendiert. Anschließend wurden serielle Verdünnungen in PBST der vereinten Überstände und der resuspendierten Komplexe auf Oxford-Agar (Profos AG) ausplattiert und für 24 h bei 37 °C inkubiert. Die Platten wurden ausgezählt und der Anteil der an den magnetischen Partikeln haftenden Listerien in Prozent der eingesetzten Zellen berechnet.

Während die Anreicherungseffizienz beim Serotyp 1/2a nahezu über den gesamten Bereich von 0 bis 1 M NaCl konstant ist, nimmt sie mit dem Serotyp 4b mit zunehmender Salzkonzentration ab.

### Experiment 7: Zellausbeute mit dem 1-Schritt-Verfahren und dem 2-Schritt-Verfahren in Abhängigkeit von der Inkubationsdauer

Kovalente Kopplung von Bio-Av-GFP-CBD_f3 an die magnetischen Partikel Dynabeads M-270 Epoxy (Dynal): Die Dynabeads wurden nach Herstellerangaben in Diglym resuspendiert und gemäß den Angaben vor der Kopplung gewaschen, äquilibriert und in 250 µl sterilem Natriumphosphat (0.1 M, pH 7.5) aufgenommen. Anschließend wurden 1 ml 3 M Ammoniumsulfatlösung in 0.1 M Natriumphosphat pH 7.4 und 1 ml Proteinlösung (StrepTag-HisTag-CBD511-f2 in 0.1 M Natriumphosphat pH 7.4) zugegeben, gemischt und ÜN bei 4 °C und dann 8 h bei RT im Rolator inkubiert. Die magnetischen Partikel wurden im Magnetfeld gesammelt der Überstand entfernt und die magnetischen Partikel 2 x in 10 mM Natriumphosphat pH 7.5, 150 mM Natriumchlorid, 0.1 % Rindersermnalbumin und 0.02 % Natriumazid für 20 min bei RT gewaschen und dann in diesem Puffer bei 4 °C gelagert. 0.5 ml einer frischen ÜN-Kultur (*L. monocytogenes* ScottA, serovar 4b; *L. moncytogenes* EGDe, serovar 1/2a) wurden auf 2 ml *Listeria* Enrichment Broth acc. FDA (Profos AG) gegeben und bis zu einer OD₆₀₀ von ca. 1 bei 37 °C gezogen (entspricht 0.5 bis 1 x 10⁹ KbE /ml). Die Kulturen wurden in PBST auf ca. 1 x 10⁴ KbE / ml verdünnt.

1-Schritt Verfahren: Zu jeweils 1 ml der Zellverdünnungen wurden 300 µg/ml der mit Bio-Av-GFP-CBD_f3 beschichteten magnetischen Partikel gegeben (Dynabeads Epoxy) und der Ansatz für 5, 10, 20, 40 und 60 min im Über-Kopf-Rolator bei RT inkubuiert. 2-Schritt Verfahren: Zu jeweils 1 ml der Zellverdünnungen wurden 5 µg und des Bio-Av-GFP-CBD_f3 Fusionsproteins gegeben und kurz gemischt. Dann wurden MagPrep-Streptavidin Partikel (Merck) zu 50 µg/ml zugegeben und die Ansätze für 5, 10, 20, 40 und 60 min im Über-Kopf-Rolator bei RT inkubuiert.

Anschließend wurden Partikel-Listerien-Komplexe im Magnetfeld an der Gefäßwandung gesammelt und der Überstand (ÜS1) in ein steriles Gefäß gegeben. Der Partikel-Listerien-Komplexe wurde in 1 ml PBST 10 min im Über-Kopf-Rolator gewaschen, im Magnetfeld an der Gefäßwandung gesammelt und der Überstand zu ÜS1 hinzugefügt. Der Komplex wurde in 1 ml PBST resuspendiert. Anschließend wurden serielle Verdünnungen in PBST der vereinten Überstände und der resuspendierten Komplexe auf Oxford-Agar (Profos AG) ausplattiert und für 24 h bei 37 °C inkubiert. Die Platten wurden ausgezählt und der Anteil der an den magnetischen Partikeln haftenden Listerien in Prozent der eingesetzten Zellen berechnet.

Mit dem 2-Schritt-Verfahren wird somit schon nach einer Inkubationszeit von 5 min die maximale Anreicherungseffizienz erreicht. Mit dem 1-Schritt-Verfahren wird erst nach 60 min dieselbe Anreicherungseffizienz erreicht wie mit dem 2-Schritt-Verfahren.

### Experiment 8: Nachweis von Listerien in Camembert mit dem 1-Schritt- und dem 2-Schritt-Verfahren

300 g Camembert aus einem Supermarkt wurden steril in 25 g Portionseinheiten aufgeteilt und in Stomacherbeuteln bei -80 °C gelagert. Eine Portionseinheit wurde auf Anwesenheit von Listeria gemäß der Vorschrift ISO: 11290-1:1996 FDAM 1 hin untersucht. Bei Abwesenheit einer Listerienköntamination wurden 5 Portionseinheiten bei RT aufgetaut und mit unterschiedlichen Mengen *L. monocytogenes* ScottA infiziert. Hierfür wurde eine ÜN-Kultur 1/5 verdünnt und bis zu einer OD₆₀₀ von ca. 1 bei 37 °C inkubiert. Anschließend wurden in sterilem PBST serielle Verdünnungen erstellt. Unter der Annahme von 0.5 bis 1 x10⁹ KbE /ml bei OD₆₀₀ von 1 wurden die Portionseinheiten mit 0, 1-10, 11-50, 50-100 und 100-500 KbE / 25 g Camembert kontaminiert und ÜN bei 4 °C gelagert. Zur exakten Bestimmung der Zellzahlen wurden Duplikate der Verdünnungen auf Oxford Agar (Profos AG) ausplattiert, die Platten 24 h bei 37°C inkubiert und ausgezählt. Zu den Portionseinheiten wurden dann 225 ml Fraser ½ Medium (Profos AG) steril zugegeben, für 1 min im Stomacher homogenisiert und bei 30 °C inkubiert. Nach einer Inkubationszeit von 4 h, 6 h und 24 h wurde je Ansatz 1 ml entnommen.

1-Schritt Verfahren: Zu 1 ml Homogenisat wurden 300 µg/ml der mit StrepTag-HisTag-CBD511-f2 beschichteten magnetischen Partikel (Dynabeads Epoxy) gegeben und der Ansatz für 20 min im Über-Kopf-Rolator bei RT inkubuiert.

2-Schritt Verfahren: Zu 1 ml Homogenisat wurden 5 µg des StrepTag-HisTag-GFP-CBD511-13 Fusionsproteins gegeben und kurz gemischt. Dann wurden MagPrep-Streptavidin Partikel (Merck) zu 50 µg/ml zugegeben und die Ansätze für 20 min im Über-Kopf-Rolator bei RT inkubuiert.

Anschließend wurden die Partikel-Listerien-Komplexe im Magnetfeld an der Gefäßwandung gesammelt und der Überstand entfernt. Der Partikel-Listerien-Komplex wurde 3 x 1 ml PBST 10 min im Über-Kopf-Rolator gewaschen, im Magnetfeld an der Gefäßwandung gesammelt und der Überstand jeweils verworfen. Die Partikel-Listerien-Komplexe wurden in 100 µl PBST resuspendiert und auf Oxford-Agar (Profos AG) ausplattiert. Nach 24 h und 48 h bei 37 °C. wurden die Platten ausgezählt und der Anteil der an den magnetischen Partikeln haftenden Listerien in Prozent der eingesetzten Zellen berechnet.Parallel wurden die kontaminierten Ansätze in Anleitung an die Norm ISO: 11290-1:1996 FDAM 1 auf Listerien hin untersucht. Hierzu wurde zu den angegebnen Zeiten 100 µl auf 10 ml Fraser-Medium (Profos AG) gegeben, 24 h bei 37 °C im Roller inkubiert und dann auf Oxford-Agar (Profos AG) ausgestrichenAlle Ansätze wurden in Quadruplets durchgeführt.

Es hat sich gezeigt, dass sowohl mit dem 1-Schritt-Verfahren, wie auch mit dem 2-Schritt-Verfahren die notwendigen Anreicherungszeiten signifikant kürzer sind als mit dem Verfahren nach ISO: 11290-1:1996, um geringe Listerienkontaminationen in Camembert nachzuweisen. Hinsichtlich der Verkürzung der Anreicherungszeit sind die Ergebnisse für das 2-Schritt-Verfahren besser als für das 1-Schritt-Verfahren.

### Experiment 9: Nachweis von Listerien in rohem Schinken und in Garnelen nach dem 1 ml und dem 10 ml 2-Schritt Verfahren

300 g roher Schinken und 300 g Garnelen aus einem Supermarkt wurden steril in 25 g Portionseinheiten aufgeteilt und in Stomacherbeuteln bei -80 °C gelagert. Jeweils eine Portionseinheit wurde auf Anwesenheit von Listeria gemäß der Vorschrift ISO: 11290-1:1996 FDAM 1 hin untersucht. Bei Abwesenheit einer Listerienkontamination wurden 5 Portionseinheiten bei RT aufgetaut und mit unterschiedlichen Mengen *L. monocytogenes* ScottA infiziert. Hierfür wurde eine ÜN-Kultur 1/5 verdünnt und bis zu einer OD₆₀₀ von ca. 1 bei 37 °C inkubiert. Anschließend wurden in sterilem PBST serielle Verdünnungen erstellt. Unter der Annahme von 0.5 bis 1 x10⁹ KbE /ml bei OD₆₀₀ von 1 wurden die Portionseinheiten mit 0, 1-10, 11-50, 50-100 und 100-500 KbE / 25 g Lebensmittel kontaminiert und ÜN bei 4 °C gelagert. Zur exakten Bestimmung der Zellzahlen wurden Duplikate der Verdünnungen auf Oxford Agar (Profos AG) ausplattiert, die Platten 24 h bei 37°C inkubiert und ausgezählt.

Zu den Portionseinheiten wurden dann 225 ml Fraser ½ Medium (Profos AG) steril zugegeben, für 1 min im Stomacher homogenisiert und bei 30 °C inkubiert. Nach einer Inkubationszeit von 4 h und 6 h wurde je Portionseinheit 2 x 1 ml und 2 x 10 ml entnommen. Zu den Homogenisaten wurde StrepTag-HisTag-GFP-CBD511-f3 Fusionsprotein zu 2,5 µg/ml zugegeben und kurz gevortext. Dann wurden MagPrep-Streptavidin Partikel (Merck) zu 100 µg/ml im 1 ml Ansatz und zu 50 µg/ml im 10 ml Ansatz zugegeben und die Ansätze für 20 min im Über-Kopf-Rolator bei RT inkubuiert. Die Partikel-Listerien-Komplexe wurden im Magnetfeld an der Gefäßwandung gesammelt und der Überstand entfernt. Der Partikel-Listerien-Komplex wurde 2 x 1 ml PBST durch mehrmaliges Aufziehen mit der Pipette gewaschen und in 100 µl PBST aufgenommen und auf Oxford-Agar (Profos AG) ausplattiert. Nach 24 h und 48 h bei 37 °C. wurden die Platten ausgezählt und der Anteil der an den magnetischen Partikeln haftenden Listerien in Prozent der eingesetzten Zellen berechnet. Parallel wurden die kontaminierten Ansätze in Anleitung an die Norm ISO: 11290-1:1996 FDAM 1 auf Listerien hin untersucht. Hierzu wurde zu den angegebnen Zeiten 100 µl auf 10 ml Fraser-Medium (Profos AG) gegeben, 24 h bei 37 °C im Roller inkubiert und dann auf Oxford-Agar (Profos AG) ausgestrichen. Alle Ansätze wurden als Duplikate durchgeführt.

Es wurde deutlich, dass durch Anreicherung mittels des 2-Schritt-Verfahrens Listerien in Schinken und Garnelen schneller nachgewiesen werden können als mit dem Verfahren nach ISO: 11290-1:1996. Eine weitere Verkürzung der Anreicherungszeit kann mit dem 10 ml 2-Schritt-Verfahren erzielt werden.

### Experiment 10: Ablösung der Listerien von den magnetischen Partikeln

0.5 ml einer frischen ÜN-Kultur von L. monocytogenes ScottA wurden auf 2 ml Listeria Enrichment Broth acc. FDA (Profos AG) gegeben und bis zu einer OD600 von ca. 1 bei 37 °C gezogen (entspricht ca. 0.5 bis 1 x 10⁹ KbE /ml). Die Kulturen wurden in PBST auf ca. 1 x 10⁴ KbE / ml verdünnt.

Zu jeweils 0.5 ml der Zellverdünnungen wurden 1 µg/ml StrepTag-HisTag-GFP-CBD511-f3 Fusionsproteins gegeben und kurz gemischt. Nach Zugabe von 50 µg/ml MagPrep-Streptavidin (Merck) wurden die Ansätze 20 min im Über-Kopf-Rolator bei RT inkubiert. Anschließend wurden die Komplexe aus magnetischen Partikeln, Fusionsprotein und Listerien im Magnetfeld an der Gefäßwandung gesammelt und der Überstand (ÜS1) in ein steriles Gefäß gegeben. Der Partikel-Protein-Listerien-Komplex wurde in 1 ml PBST 10 min im Über-Kopf-Rolator gewaschen, im Magnetfeld an der Gefäßwandung gesammelt und der Überstand zu ÜS1 hinzugefügt.

Die Hälfte der Ansätze wurden in 100 µl PBST resuspendiert, die andere Hälfte wurde in 100 µl 50 mM Natriumphosphat pH 11 resuspendiert. Nach 5 min bei RT wurden die magnetischen Partikel abgezogen und die Überstände zu 400 µl PBST gegeben. Die magnetischen Partikel wurden in 0.5 ml resuspendiert.

Serielle Verdünnungen mit PBST der Überstände und der resuspendierten magnetischen Partikel wurden auf Oxford-Agar (Profos AG) ausplattiert und für 24 h bei 37 °C inkubiert. Die Platten wurden ausgezählt und der Anteil der an den magnetischen Partikeln haftenden Listerien in Prozent der insgesamt wieder gefundenen Listerien berechnet. 90 % der über die Zellbindedomäne an die magnetischen Partikel gebundenen Listerien wurden mit dem pH 11 Puffer wieder abgelöst.

### Experiment 11: Anreicherung von Listerien und Nachweis über PCR

0.5 ml einer frischen ÜN-Kultur von L. monocytogenes ScottA wurden auf 2 ml Listeria Enrichment Broth acc. FDA (Profos AG) gegeben und bis zu einer OD600 von ca. 1 bei 37 °C gezogen (entspricht ca. 0.5 bis 1x10⁹ KbE/ml). Die Kultur wurde in *Listeria* Enrichment Broth acc. FDA (Profos AG) auf ca. 1 x 10⁶ und 1 x 10⁵ KbE / ml verdünnt.

Je Zellverdünnung in *Listeria* Enrichment Broth acc. FDA (Profos AG) wurden 14 Ansätze ä 1 ml vorgelegt. Davon wurden 4 Ansätze zentrifugiert (5 min 13000 rpm in der Tischzentrifuge), die Überstände wurden verworfen, die Zellen 1 x in PBST gewaschen und pelletiert. Die Zellpellets wurden bis zur weiteren Verarbeitung auf Eis aufbewahrt.

Aus 10 Ansätzen wurden die Zellen über das 2-Schritt-Verfahren isoliert: Zu jeweils 1 ml der Zellverdünnungen wurden 20 µg des StrepTag-HisTaG-GFP-CBD511-f2 Fusionsproteins gegeben und kurz gemischt. Dann wurden Streptavidin-Magnetic-Particles (Roche) zu 100 µg/ml zugegeben und die Ansätze für 20 min im Über-Kopf-Rolator bei RT inkubuiert. Anschließend wurden die Partikel-Listerien-Komplexe im Magnetfeld an der Gefäßwandung gesammelt, der Überstand abgezogen und verworfen. Der Partikel-Listerien-Komplex wurde in 1 ml PBST 10 min im Über-Kopf-Rolator gewaschen, im Magnetfeld an der Gefäßwandung gesammelt, der Überstand verworfen.

Der Partikel-Listerien-Komplex von je 2 Ansätzen wurde in 1 ml PBST aufgenommen. Davon wurden serielle Verdünnungen auf Oxford-Agar (Profos AG) ausplattiert und für 24 h bei 37 °C inkubiert. Zusätzlich wurden serielle Verdünnungen der Ausgangszellverdünnungen mit geschätzten 10⁶ und 10⁵ KbE/ml auf Oxford-Agar (Profos AG) ausplattiert und für 24 h bei 37 °C inkubiert. Die Platten wurden ausgezählt und die tatsächliche Zellzahl (1.4 x 10⁵ und 1,4 x 10⁶ KbE/ml) und der Anteil der an den magnetischen Partikeln haftenden Listerien in Prozent der eingesetzten Zellen (85-95 %) berechnet.

Der Partikel-Listerien-Komplex von 4 Ansätzen wurde in 20 µl 150 mM Natriumphosphat pH 11 aufgenommen, 15 min bei RT inkubiert, dann wurden die magnetischen Partikel im Magnetfeld an der Gefäßwandung gesammelt und der Überstand quantitativ auf 10 µl 150 mM Natriumphosphat pH 6 gegeben (Ansätze A). Der Partikel-Listerien-Komplex der 4 verbleibenden Ansätze wurde in 20 µl PBST resuspendiert (Absätze B). Die jeweils 4 Ansätze der abzentrifugierten Zellen wurden ebenfalls in 20 µl PBST resuspendiert (Ansätze C).

Zu je 2 Ansätzen A, B und C wurden 10 µl PBST gegeben, zu den jeweils verbliebenen 2 Ansätzen 10 µl PBST mit Ply511 (2 µg/ml) und bei 40 °C 5 -10 min inkubiert. Dann wurde zu allen 4 Ansätzen 5 µl Proteinase K (1 mg/ml PBST) gegeben, 5 min bei 56 °C und dann 5 min bei 94°C inkubiert. Die magnetischen Partikel der Ansätze B wurden im Magnetfeld abgezogen und von allen Ansätzen A, B und C wurden 5 µl in eine PCR-Reaktion nach Aznar & Alarcon (Aznar R & Alarcon B, 2002, On the specificity of PCR detection of Listeria moncytogenes in food, System. Appl. Microbiol. 25, 109-119) eingesetzt und anschließend die Reaktionsprodukte anschließend über ein 1 % Agarosegel aufgetrennt.

Es hat sich gezeigt, dass in allen 3 Fällen (Zentrifugation, 2-Schritt-Verfahren mit Aufbruch an den magnetischen Partikeln und 2-Schritt-Verfahren mit Ablösung von den magnetischen Partikeln) die Zugabe von Endolysin Ply511 zum Zellaufbruch die Sensitivität des Nachweises erheblich verbessert. Während mit der Lyse der Listerien an den magnetischen Partikeln die gleiche Sensitivität erreicht wird wie wenn die Zellen abzentrifugiert werden, ist die Signalstärke der PCR geringer, wenn die Zellen vor Aufbruch von den magnetischen Partikeln durch pH 11 Puffer abgelöst werden.

### Experiment 12: Nachweis von Listerien aus Frankfurter Würstchen und Mozzarella

Zu jeweils 25 g Frankfurter bzw. Mozzarella wurden je 225 ml FDA-Medium hinzugegeben und die Portionen steril in Stomacherbeuteln homogenisiert. Die Proben wurden über Nacht bei 30 °C inkubiert. Vor dem Listeriennachweis wurden die Proben jeweils mit 1/10 Volumen PBST gepuffert.

1-Schritt Verfahren: Zu 1 ml Homogenisat wurden 300 µg/ml der mit Bio-Av-GFP-CBD511_f3 beschichteten magnetischen Partikel (Dynabeads M270 Epoxy) gegeben und der Ansatz für 20 min im Über-Kopf-Rolator bei RT inkubuiert.

2-Schritt Verfahren: Zu 1 ml Homogenisat wurden je 0.5, 2, 5 oder 10 µg des Bio-Av-GFP-CBD511_f3 Fusionsproteins gegeben und kurz gemischt. Dann wurden MagPrep-Streptavidin Partikel (Merck) zu 50 µg/ml zugegeben und die Ansätze für 20 min im Über-Kopf-Rolator bei RT inkubuiert.

Anschließend wurden die Partikel-Listerien-Komplexe im Magnetfeld an der Gefäßwandung gesammelt und der Überstand entfernt. Der Partikel-Listerien-Komplex wurde 1 x 1 ml PBST 10 min im Über-Kopf-Rolator gewaschen, im Magnetfeld an der Gefäßwandung gesammelt und der Überstand jeweils verworfen. Die Partikel-Listerien-Komplexe wurden in 100 µl PBST resuspendiert und auf Oxford-Agar (Profos AG) ausplattiert. Nach 24 h bei 37 °C. wurden die Platten ausgezählt und der Anteil der an den magnetischen Partikeln haftenden Listerien in Prozent der eingesetzten Zellen berechnet. Alle Ansätze wurden 2fach durchgeführt.

Es hat sich gezeigt, dass sowohl aus Frankfurter Würstchen als auch aus Mozzarella mit Hilfe des Bio-Av-GFP-CBD511_f3 Fusionsproteins Listerien isoliert werden konnten. Bei Mozzarella gelingt dies mit dem Stamm EGDe wesentlich besser als mit ScottA. Bei den Lebensmitteln werden etwas höhere Konzentrationen an Protein eingesetzt, um eine hohe Bindungseffizienz zu erreichen. Während das 1-Schritt-Verfahren bei Frankfurter Würstchen und dem Stamm EGDe eine hohe Bindungseffizienz aufweist, ist bei allen anderen Bedingungen das 2-Schritt-Verfahren besser geeignet.

## Patentansprüche

1. Polypeptidfragment von Endolysin Ply511, **dadurch** charakterisiert, dass es Listerien bindet, aber keine Zellwand hydrolysierende enzymatische Aktivität aufweist, und das Polypeptidfragment eine Aminosäuresequenz aufweist, die bezogen auf die Volllängensequenz gemäß SEQ ID NO:2 mindestens der Aminosäuresequenz von 180-341 und höchstens der Aminosäuresequenz von 116-341 entspricht.

2. Polypeptidfragment nach Anspruch 1 mit einer Aminosäuresequenz gemäß SEQ ID NO:4, 6 oder 8.

3. Polypeptidfragment nach Anspruch 1 oder 2, wobei das Polypeptidfragment ferner einen Affinitäts-Tag, oder ein Spacermolekül gegebenenfalls mit einem Affinitäts-Tag oder ein Biotin aufweist.

4. Polypeptidfragment nach Anspruch 3, wobei der Affinitäts-Tag ein His-Tag, Strep-Tag, Avi-Tag oder eine Biotinylierungsdomäne ist.

5. Polypeptidfragment nach Anspruch 3, wobei das Spacermolekül GFP, MBP oder eine Biotinylierungsdomäne ist.

6. Nukleinsäuremolekül mit einer Sequenz kodierend für ein Polypeptidfragment nach einem der Ansprüche 1 bis 5.

7. Nukleinsäuremolekül nach Anspruch 6 mit einer Sequenz gemäß SEQ ID NO:3, 5 oder 7.

8. Verfahren zur Anreicherung und/oder Entfernung von Listerien aus einer Probe, umfassend die Schritte
a) Inkubation oder in Kontakt bringen einer Probe mit einem Polypeptid gemäß einem der Ansprüche 1 bis 5, das unspezifisch oder gerichtet, an einem festen Träger immobilisiert ist,
b) Trennen des Träger-Polypeptid-Listerien-Komplexes von der Probe.

9. Verfahren nach Anspruch 8, ferner umfassend nach Schritt b) den Schritt c) Abwaschen unspezifisch am Träger-Polypeptid-Listerien-Komplex haftender Bestandteile der Probe.

10. Verfahren nach Anspruch 8 oder 9, wobei die Schritte a) und b) in einem Chromatographiesäulen-Durchflussverfahren durchgeführt werden.

11. Verfahren nach einem der Ansprüche 8 bis 10, wobei der feste Träger Cellulose, Filtrationsmedien, Glaspartikel, Magnetpartikel, Zentrifugations-, Sedimentationsmaterialien oder Füllmaterialien für Chromatographiesäulen sind.

12. Verfahren zur Anreicherung und/oder Entfernung von Listerien aus einer Probe, umfassend die Schritte
a) Inkubation oder in Kontakt bringen einer Probe mit einem Polypeptid gemäß einem der Ansprüche 3 bis 5,
b) in Kontakt bringen und Inkubation von Listerien-Polypeptid-Komplex mit einem Träger, der mit dem jeweiligen Bindepartner des Polypeptids oder einer chemischen Gruppe beschichtet ist,
c) Trennen des Träger-Polypeptid-Listerien-Komplexes von der Probe.

13. Verfahren nach Anspruch 12, ferner umfassend nach Schritt c) den Schritt d) Abwaschen unspezifisch am Träger-Polypeptid-Listerien-Komplex haftender Bestandteile der Probe.

14. Verfahren zum Nachweis von Listerien in einer Probe, umfassend ferner nach Schritt b) gemäß Anspruch 8 oder nach Schritt c) gemäß Anspruch 12 den Schritt: Nachweis der Listerien.

15. Kit, umfassend einen Träger, an den Polypeptidfragmente nach einem der Ansprüche 1-5 immobilisiert sind und Waschpuffer, Ablösepuffer und/oder Zellaufbruchspuffer.

16. Kit, umfassend ein Polypeptidfragment nach einem der Ansprüche 3-5, einen Träger beschichtet mit dem jeweiligen Bindepartner des Affinitäts-Tags, des Spacermoleküls oder der Biotinylierungsdomäne und Waschpuffer, Ablösepuffer und/oder Zellaufbruchspuffer.

## Claims

1. Polypeptide fragment of endolysin PLy511 **characterised in that** it binds listeria, but does not exhibit any cell wall hydrolysing enzymatic activity and the polypeptide fragment has an amino acid sequence, which corresponds to at least the amino acid sequence of 180-341 and at most to the amino acid sequence of 116-341 referring to the full length sequence according to SEQ ID NO: 2.

2. Polypeptide fragment according to claim 1 having an amino acid sequence according to SEQ ID NO: 4, 6 or 8.

3. Polypeptide fragment according to claim 1 or 2, wherein the polypeptide fragment further exhibit an affinity tag or a spacer molecule, optional with an affinity tag or a biotin.

4. Polypeptide fragment according to claim 3, wherein the affinity tag is a His-tag, Strep-tag, Avi-tag or a biotinylation domain.

5. Polypeptide fragment according to claim 3, wherein the spacer molecule is GFP, MBP or a biotinylation domain.

6. Nucleic acid molecule having a sequence coding for a polypeptide fragment according to anyone of claims 1 to 5.

7. Nucleic acid molecule according to claim 6 having a sequence according to SEQ ID NO: 3, 5 or 7.

8. Method for enrichment and/or removal of listeria from a sample, the method comprising the steps of
a) incubating or bringing a sample into contact with a polypeptide according to anyone of claims 1 to 5, which is unspecifically or directedly immobilized to a permanent carrier,
b) separating of the carrier-polypeptide-listeria complex from the sample.

9. Method according to claim 8, further comprising after step b) the step c) washing away parts of the sample unspecifically adhering to the carrier-polypeptide-listeria-complex.

10. Method according to claim 8 or 9, wherein the steps a) and b) are performed in a chromatography column flow-through method.

11. Method according to anyone of claims 8 to 10, wherein the permanent carrier is cellulose, filtration media, glass particles, magnetic particles, centrifugations-, sedimentations materials or filling materials for chromatography columns.

12. Method for the enrichment and/or removal of listeria from a sample, the method comprising the steps
a) incubating or bringing a sample into contact with a polypeptide according to anyone of claims 3 to 5,
b) bringing into contact and incubating of listeria-polypeptide-complex with a carrier, which is coated with the respective binding partner of the polypeptide or a chemical group,
c) separating of the carrier-polypeptide-listeria-complex from the sample.

13. Method according to claim 12, further comprising after step c) the step d) washing away compounds of the sample unspecifically adhering to the carrier-polypeptide-listeria-complex.

14. Method for the detection of listeria in a sample, the method further comprising after step b) according to claim 8 or after step c) according to claim 12 the step of: detection of the listeria.

15. Kit comprising a carrier on which polypeptide fragments according to anyone of claims 1-5 are immobilized and washing buffer, detaching buffer and/or cell cracking buffer.

16. Kit comprising a polypeptide fragment according to anyone of claims 3-5, a carrier coated with the respective binding partner of the affinity tag, the spacer molecule or the biotinylation domain and washing buffer, detaching buffer and/or cell cracking buffer.

## Revendications

1. Fragment de polypeptide d'endolysine Ply511, **caractérisé en ce qu'**il lie les bactéries Listeria, mais ne présente pas d'activité enzymatique hydrolysant les parois cellulaires, et **en ce que** le fragment de polypeptide présente une séquence d'acides aminés qui correspond, par rapport à la séquence de longueur complète selon la SEQ ID N°2, au moins à la séquence d'acides aminés 180-341 et au plus à la séquence d'acides aminés 116-341.

2. Fragment de polypeptide selon la revendication 1 comprenant une séquence d'acides aminés selon la SEQ ID N°4,6 ou 8.

3. Fragment de polypeptide selon la revendication 1 ou 2, dans lequel le fragment de polypeptide présente en outre un tag d'affinité, ou une molécule espaceur, le cas échéant avec un tag d'affinité, ou une biotine.

4. Fragment de polypeptide selon la revendication 3, dans lequel le tag d'affinité est un tag His, un tag Strep, un tag Avi ou un domaine de biotinylation.

5. Fragment de polypeptide selon la revendication 3, dans lequel la molécule espaceur est la GFP, MBP ou un domaine de biotinylation.

6. Molécule d'acide nucléique comprenant une séquence codant pour un fragment de polypeptide selon une des revendications 1 à 5.

7. Molécule d'acide nucléique selon la revendication 6 comprenant une séquence selon la SEQ ID N°3, 5 ou 7.

8. Procédé d'enrichissement et/ou d'élimination des bactéries Listeria d'un échantillon, comprenant les étapes de :
a) incubation ou mise en contact d'un échantillon avec un polypeptide selon une des revendications 1 à 5, qui est immobilisé aspécifiquement ou de manière dirigée sur un support solide,
b) séparation du complexe support - polypeptide - bactéries Listeria et de l'échantillon.

9. Procédé selon la revendication 8, comprenant en outre, après l'étape b), l'étape
c) lavage des éléments de l'échantillon qui adhèrent de façon non spécifique au complexe support - polypeptide - bactéries Listeria.

10. Procédé selon la revendication 8 ou 9, dans lequel les étapes a) et b) sont réalisées dans un procédé de passage au-travers de colonnes chromatographiques.

11. Procédé selon l'une des revendications 8 à 10, dans lequel le support solide est constitué par la cellulose, des milieux de filtration, des particules de verre, des particules magnétiques, des matériaux de centrifugation, de sédimentation ou des charges pour les colonnes chromatographiques.

12. Procédé d'enrichissement et/ou d'élimination des bactéries Listeria d'un échantillon, comprenant les étapes de :
a) incubation ou mise en contact d'un échantillon avec un polypeptide selon l'une des revendications 3 à 5,
b) mise en contact et incubation du complexe bactéries Listeria - polypeptide avec un support, qui est recouvert du partenaire de liaison respectif du polypeptide ou d'un groupe chimique,
c) séparation du complexe support - polypeptide - bactéries Listeria et de l'échantillon.

13. Procédé selon la revendication 12, comprenant en outre, après l'étape c), l'étape d)
lavage des éléments de l'échantillon qui adhèrent de façon non spécifique au complexe support - polypeptide - bactéries Listeria.

14. Procédé de détection des bactéries Listeria dans un échantillon, comprenant en outre, après l'étape b) selon la revendication 8 ou après l'étape c) selon la revendication 12, l'étape de détection des bactéries Listeria.

15. Kit, comprenant un support, sur lequel sont immobilisés des fragments de polypeptide selon l'une des revendications 1 à 5, et du tampon de lavage, du tampon de transfert et/ou du tampon de lyse cellulaire.

16. Kit, comprenant un fragment de polypeptide selon l'une des revendications 3 à 5, un support recouvert du partenaire de liaison respectif du tag d'affinité, de la molécule espaceur ou du domaine de biotinylation, et du tampon de lavage, du tampon de transfert et/ou du tampon de lyse cellulaire.
